# EUROPEAN PATENT SPECIFICATION

(11) **EP 3 600 278 B1**
(45) Date of publication and mention of the grant of the patent: **27.01.2021**
(21) Application number: 18715625.2
(22) Date of filing: 29.03.2018
(51) Int. Cl.: A61K 31/19, A61K 31/40, A61P 9/10

(54) **STATIN FOR PREVENTION/REDUCTION OF ISCHEMIA-RELATED DAMAGE**
STATIN ZUR VERHINDERUNG/VERRINGERUNG VON BESCHÄDIGUNG IM ZUSAMMENHANG MIT ISCHÄMIE
STATINE POUR LA PRÉVENTION/RÉDUCTION DE DOMMAGE DU À L'ISCHÉMIE

(30) Priority: 31.03.2017 EP 17164137; 26.05.2017 ES 201730736
(43) Date of publication of application: 05.02.2020
(73) Proprietor: Fundació Institut de Recerca de l'Hospital de la Santa creu i Sant Pau, 08025 Barcelona (ES); Gendiag, S.L., 08013 Barcelona (ES)
(72) Inventor: BADIMON MAESTRO, Lina, 08025 Barcelona (ES); VILAHUR GARCIA, Gemma, 08025 Barcelona (ES)
(74) Representative: Hoffmann Eitle
(86) International application number: PCT/EP2018/058158
(87) International publication number: WO 2018/178275

(56) References cited:
- WO-A1-2015/181216
- SEBASTIAN WOLFRUM ET AL: "Simvastatin Acutely Reduces Myocardial Reperfusion Injury In Vivo by Activating the Phosphatidylinositide 3-Kinase/Akt Pathway", JOURNAL OF CARDIOVASCULAR PHARMACOLOGY, vol. 44, no. 3, 2004, pages 348-355, XP055158989, ISSN: 0160-2446, DOI: 10.1097/01.fjc.0000137162.14735.30
- XIANJIN DU ET AL: "Postconditioning with rosuvastatin reduces myocardial ischemia-reperfusion injury by inhibiting high mobility group box 1 protein expression", EXPERIMENTAL AND THERAPEUTIC MEDICINE, vol. 7, 2014, pages 117-120, XP055159050, ISSN: 1792-0981, DOI: 10.3892/etm.2013.1362
- NESIC ZORICA ET AL: "Acute protective effects of different doses of simvastatin in the rat model of renal ischemia-reperfusion injury", ACTA VETERINARIA (BEOGRAD), vol. 58, no. 5-6, 2008, pages 413-427, XP055159056, ISSN: 0567-8315, DOI: 10.2298/AVB0806413N
- KEFEI WU ET AL: "Atorvastatin treatment attenuates renal injury in an experimental model of ischemia-reperfusion in", BMC NEPHROLOGY, vol. 15, 14, 2014, pages 1-10, XP021172175, ISSN: 1471-2369, DOI: 10.1186/1471-2369-15-14

## Description

### FIELD OF THE INVENTION

The present disclosure relates, in general, to the prevention and/or reduction of ischemia-related damage. More specifically, the present disclosure relates to compounds for use in the prevention and/or reduction of ischemia-related damage.

### BACKGROUND OF THE INVENTION

Ischemia occurs when blood flow to an area of cells is insufficient to support their regular metabolic activity. As a consequence, the affected tissue or organ may suffer severe damage. Restricted blood flow leads to the build-up of metabolic waste products and insufficiency of cellular energy, which results in breakdown of cellular functions and eventually in cellular necrosis. In addition, ischemia induces apoptosis and local and systemic inflammation, resulting in additional tissue damage.

Reperfusion (i.e. restoration of blood supply to the affected organ or tissue after a period of ischemia) leads to further damage due to inflammation and oxidative stress (the so-called reperfusion damage). Thus, although reperfusion salvages ischemic tissue that would otherwise die due to accumulating ischemic damage, restoring blood flow to the affected tissue exacerbates injury compared to the damage at the onset of reperfusion thereby increasing the final size of infarction. The final infarct size is determined by both the damage induced during ischemia and the damage produced during reperfusion which, in turn, is associated to the ischemia. Nevertheless, to limit the ischemic damage during the period of restricted blood flow, immediate intervention and measures to ensure reperfusion of the ischemic organ or tissue is imperative.

An example of ischemia is the impaired perfusion of cardiac tissue. This results in a loss of the heart's ability to function properly as the tissue becomes oxygen- and energy-deprived. Cardiac injury is directly related to the duration of the ischemic period. Current interventions for improving blood flow to damaged heart tissue are mostly invasive, including percutaneous coronary interventions with or without stent placement, coronary bypass surgery, angioplasty, and endarterectomy. However, there are high risks associated with such invasive procedures.

On a molecular level, the continuous deficiency of oxygen during ischemia shifts cardiac metabolism toward anaerobic glycolysis which produces a decrease in cell pH. To buffer this accumulation of hydrogen ions, the Na⁺/H⁺ exchanger excretes excess hydrogen ions, which produces a large influx of sodium ions and Ca²⁺ overload. Ischemia also depletes cellular ATP which inactivates ATPases (e.g., Na⁺/K⁺ ATPase), reduces active Ca²⁺ efflux, and limits the reuptake of calcium by the endoplasmic reticulum (ER), thereby producing calcium overload in the cell. These changes are accompanied by opening of the mitochondrial permeability transition (MPT) pore, which dissipates mitochondrial membrane potential and further impairs ATP production and also triggers cell death. As such, in the heart, these cellular changes are accompanied by the activation of intracellular proteases (e.g., calpains) which damage myofibrils and produce hypercontracture and contracture band necrosis. These alterations and thus the degree of tissue injury vary in extent depending on the magnitude and the duration of the ischemic period.

There is an interaction between ischemic damage and reperfusion damage. The greatest the ischemic damage is the higher the impact in reperfusion damage. For example, during ischemia there is a decrease in intracellular pH, which activates the Na⁺/H⁺ exchange system with extrusion of H⁺ from the cell in exchange for Na⁺. This leads to an increase in intracellular sodium, which continues during reperfusion. The normal Na⁺/K⁺ ATPase system, which extrudes sodium, is inhibited in this acid medium. This results in activation of the Na⁺/Ca²⁺ exchanger leading to a buildup of intracellular calcium and eventually cell death. Other biochemical events that occur during ischemia and also contribute to ischemic/reperfusion damage are the induction of a systemic and local inflammatory response and an increase in the susceptibility to platelet activation. The inflammatory response that occurs upon reperfusion is a coordinated interplay between the circulating leukocytes (mainly neutrophils) and the vascular endothelium as well as the cardiac resident macrophages and mast cells. The reintroduction of O₂ results in a respiratory burst phenomenon with the production of toxic oxygen-derived free radicals, such as superoxide anion (O₂⁻), H₂O₂ and hydroxyl radical (OH•). This generation of oxidants in excess of the capacity of the endogenous scavenging systems results in complement activation, production of leukotriene (LT)B4 and platelet activating factor (PAF), mobilization of P-selectin from the Weibel-Palade bodies, as well as synthesis of E-selectin and intracellular adhesion molecule (ICAM)-1 through the activation of nuclear transcription factors (e.g., NF-κB). There is also a change in the ionic composition of the cytoplasm with increased calcium. For example, in cardiac myocytes there is uncoupling of oxidative phosphorylation and tension generation due to energy wasting mechanisms, such as increased activation of calcium transport at the inner mitochondrial membrane and sarcoplasmic reticulum. This creates a vicious cycle where calcium causes an increase in xanthine oxidase, promoting the production of oxygen radicals, resulting in further calcium influx. The outpouring of cytokines, PAF3 and LTB4 promotes leukocyte recruitment leading to local and eventually systemic inflammation.

Despite an improved understanding of the pathophysiology of the molecular events that occur during ischemia, effective therapies to prevent or minimize ischemia-related damage are still not available. Thus, there is an unmet need for improved ways to prevent or reduce ischemia-related damage, in particular insofar as the ischemia-related damage is ischemia damage. Moreover, there is a need for ways to prevent/reduce ischemia-related damage that are less risky for the patient, less cost intensive and/or result in less ischemia-related damage, in particular with respect to the size of the infarction caused by ischemia, tissue damage due to cell death (necrosis and/or apoptosis) triggered by ischemia, tissue damage caused by the inflammatory reaction that occurs during ischemia within the ischemic tissue/organ, damage caused by the systemic inflammatory response triggered by ischemia and/or scar formation in the post-ischemic tissue. Moreover, there is a need for ways to prevent/reduce ischemia-related damage that are less risky for the patient, less cost intensive and/or result in less ischemia-related damage, in particular with respect to damage to myofibrils caused by intracellular proteases. In particular, there is a need for means that allow achieving several of these goals simultaneously by a single treatment.

The present disclosure meets these above needs and solves the above-described problems by the different aspects and embodiments described below.

### SUMMARY OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

The authors of the present disclosure have found that intravenous administration of a statin (e.g. atorvastatin or the β-hydroxy acid form of simvastatin) after the onset of ischemia reduces ischemia-related damage. In particular, they have found in small and large animal models (rodents and pigs) that the administration of this treatment regime early after myocardial ischemia induces heart protection by limiting ischemia-related damage.

As can be seen from the enclosed examples, the intravenous infusion of the statin atorvastatin early after the onset of ischemia in a swine model of closed-chest coronary balloon occlusion limited myocardial damage induced by ischemia, reduced cell death execution in the ischemic myocardium and limited the inflammatory response within the infarcted myocardial tissue. Although the inventors do not wish to be bound by any theory, observations made by the present inventors suggest that the protective effects may involve RhoA inhibition and AMPK (AMP-activated protein kinase) activation. These observations in a swine model that closely resembles the human physiology have a high translational potential to be implemented in human patients with the aim to prevent/reduce ischemia-related damage by intravenous statin administration early after the onset of ischemia.

Preferably, the present disclosure is defined as follows:
[1] A compound for use in the prevention and/or reduction of ischemia-related damage in a subject, wherein said compound is a statin, wherein said compound is to be administered to said subject by intravenous administration after the onset of ischemia, but prior to reperfusion, wherein the compound is to be administered as early as possible after the onset of ischemia.
[2] The compound for use according to [1], wherein ischemia-related damage is the damage caused by ischemia during the ischemic period after the onset of ischemia, but prior to reperfusion.
[3] The compound for use according to any of [1] or [2], wherein the compound is to be administered in a single dose.
[4] The compound for use according to any of [1] to [3], wherein the compound is to be administered not more than 60 minutes after the onset of ischemia, preferably not more than 45 minutes after the onset of ischemia, more preferably not more than 30 minutes after the onset of ischemia, more preferably not more than 20 minutes after the onset of ischemia, more preferably not more than 15 minutes after the onset of ischemia.
[5] The compound for use according to any of [1] to [4], wherein the compound is to be administered at least 15 minutes prior to reperfusion, preferably at least 20 minutes prior to reperfusion, more preferably at least 30 minutes prior to reperfusion, more preferably at least 45 minutes prior to reperfusion.
[6] The compound for use according to any of [1] to [5], wherein the compound is selected from the compounds listed in Table 1 wherein, preferably, the compound is selected from the group consisting of atorvastatin, the β-hydroxy acid form of simvastatin, simvastatin, cerivastatin, fluvastatin, lovastatin, mevastatin, pitavastatin, pravastatin and rosuvastatin, wherein, more preferably, the compound is atorvastatin (formula I) or the β-hydroxy acid form of simvastatin of formula II, wherein, most preferably, the compound is atorvastatin (formula I) or the β-hydroxy acid form of simvastatin of formula III: 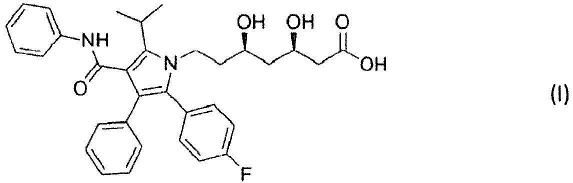 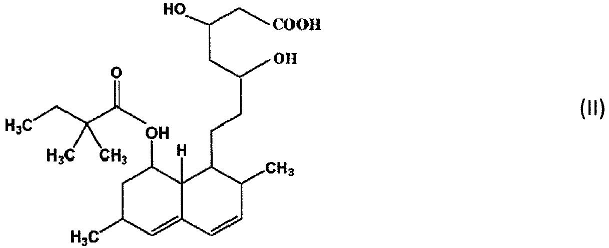 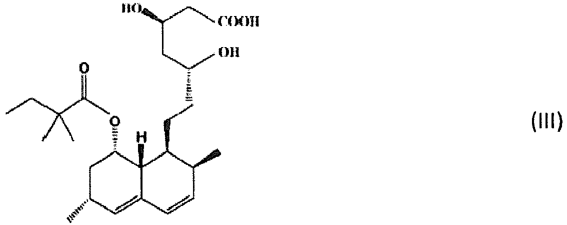
[7] The compound for use according to any of [1] to [6], wherein the compound is to be administered at a dosage in the range of from 0.1 to 1 mg/kg body weight, preferably from 0.2 to 0.8 mg/kg body weight.
[8] The compound for use according to any of [1] to [7], wherein the ischemia is produced in a tissue or organ selected from the group consisting of heart, brain, kidney, intestine, pancreas, liver, lung, skeletal muscle and combinations thereof, wherein, preferably, the ischemia is produced in the heart.
[9] The compound for use according to any of [1] to [8], wherein ischemia-related damage is due to an ischemia caused by atherosclerosis, thrombosis, thrombo-embolism, lipid-embolism, bleeding, stent, surgery, angioplasty, bypass surgery, organ transplantation, stress-induced cardiomyopathy (Takotsubo syndrome), vasoconstriction, ST-elevated myocardial infarction, non-ST-elevated myocardial infarction, unstable angina, or a combination of two or more of these, wherein, preferably, the ischemia is related to ST-elevated myocardial infarction, non-ST-elevated myocardial infarction or unstable angina.
[10] The compound for use according to any of [1] to [9], wherein the ischemia-related damage comprises or consists of (preferably consists of) at least one damage selected from the group consisting of myocyte cell death (preferably through necrosis and apoptosis, more preferably through necrosis), damage caused by intracellular pH acidification due to ischemia and/or damage caused by an inflammatory response due to ischemia that, preferably, is triggered by ischemia (and preferably further amplified during reperfusion), wherein, more preferably, said ischemia-related damage consists of damage caused by intracellular pH acidification due to ischemia.
[11] The compound for use according to any of [1] to [10], wherein the ischemia-related damage comprises or consists of (preferably consists of) damage to myofibrils caused by intracellular proteases (which causes hypercontracture and/or contracture band necrosis).
[12] The compound for use according to any of [1] to [11], wherein the prevention/reduction of ischemia-related damage comprises or consists of a reduction of the infarct size caused by ischemia, preferably a reduction of myocardial damage.
[13] The compound for use according to any of [1] to [12], wherein the prevention/reduction of ischemia-related damage leads to a reduction of tissue damage due to cell death triggered by ischemia/reperfusion, preferably due to cell death triggered by ischemia.
[14] The compound for use according to any of [1] to [13], wherein the prevention/reduction of ischemia-related damage comprises or consists of a prevention or reduction of the inflammatory response in the ischemic tissue/organ.
[15] The compound for use according to any of [1] to [14], wherein the prevention/reduction of ischemia-related damage comprises or consists of the reduction of the inflammatory response in blood cells, preferably in leukocytes, triggered by ischemia and/or reperfusion, preferably triggered by ischemia.
[16] The compound for use according to any of [1] to [15], wherein the prevention/reduction of ischemia-related damage comprises or consists of a reduction in scar size in the post-ischemic tissue, preferably in post-ischemic cardiac tissue.
[17] The compound for use according to any of [1] to [16], wherein the subject is a human.
[18] The compound for use according to any of [1] to [17], wherein the subject suffers from dyslipidemia, wherein, preferably, said dyslipidemia is hypercholesterolemia.
[19] The compound for use according to any of [1] to [18], wherein the statin is not administered at and/or after reperfusion.
[20] The compound for use according to any of [1] to [19], wherein the statin is not administered during reperfusion.
[21] The compound for use according to any of [1] to [20], wherein the compound is to be administered at least 15, preferably at least 20 minutes, more preferably at least 30 minutes, even more preferably at least 45 minutes prior to reperfusion.
[22] The compound for use according to any of [1] to [21], wherein the administration reduces the level of at least one cardiac damage-related marker selected from the group consisting of IMA (ischemic modified albumin), CFABP (cardiac fatty acid binding protein) and myoglobin, wherein, preferably, the administration reduces the level of the cardiac damage-related markers IMA (ischemic modified albumin), CFABP (cardiac fatty acid binding protein) and myoglobin.
[23] The compound for use according to any of [1] to [22], wherein said administration results in a reduced size of myocardial infarction.
[24] The compound for use according to any of [1] to [23], wherein the statin inhibits RhoA activation.
[25] The compound for use according to any of [1] to [24], wherein said administration activates AMPK (by phosphorylation of AMP-activated protein kinase).
[26] The compound for use according to any of [1] to [25], wherein said administration results in a reduction in the cellular level of phosphorylated (active) p53.
[27] The compound for use according to any of [1] to [26], wherein said administration results in a reduction in the cellular level of cleaved (active) caspase-3.
[28] The compound for use according to any of [1] to [27], wherein said administration results in a reduction in the cellular level of MCP-1/CCL2 at the mRNA and/or at the protein level.
[29] A pharmaceutical composition comprising
   - the compound for use according to any of [1] to [28] and
   - a pharmaceutically acceptable excipient or carrier
   for use in the prevention/reduction of ischemia-related damage in a subject. In preferred embodiments of said pharmaceutical composition, said compound, said prevention/reduction, said ischemia-related damage and/or said subject are as defined in any of [2] to [28].
[30] Use of a compound for the manufacture of a medicament for the prevention/reduction of ischemia-related damage in a subject,
   wherein said compound is a statin,
   wherein said compound is to be administered to said subject by intravenous administration after the onset of ischemia, but prior to reperfusion,
   and wherein said compound is to be administered as early as possible after the onset of ischemia.

In preferred embodiments of said use, said compound is as defined in any of [2] to [28].
[31] A method of treatment comprising the step of administering to a subject suffering from ischemia-related damage a pharmaceutically effective amount of a compound, wherein said compound is a statin,
wherein said compound is administered to said subject by intravenous administration after the onset of ischemia, but prior to reperfusion,
and wherein said compound is administered as early as possible after the onset of ischemia.

In preferred embodiments of said method of treatment, said compound is as defined in any of [2] to [28].

### BRIEF DESCRIPTION OF THE DRAWINGS

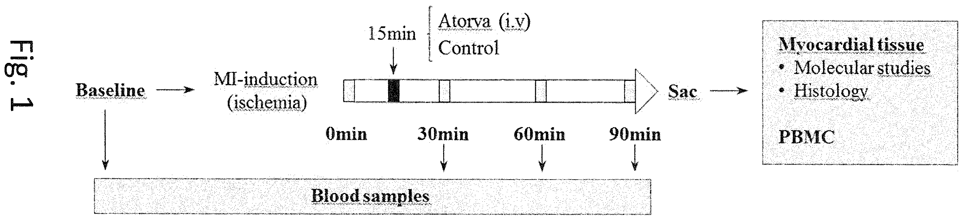

**Figure 1** is a flow chart showing the experimental protocol underlying Example 1. MI: myocardial infarction. Sac: Sacrifice; PBMC: peripheral blood mononuclear cells (a type of leukocyte); atorva: atorvastatin

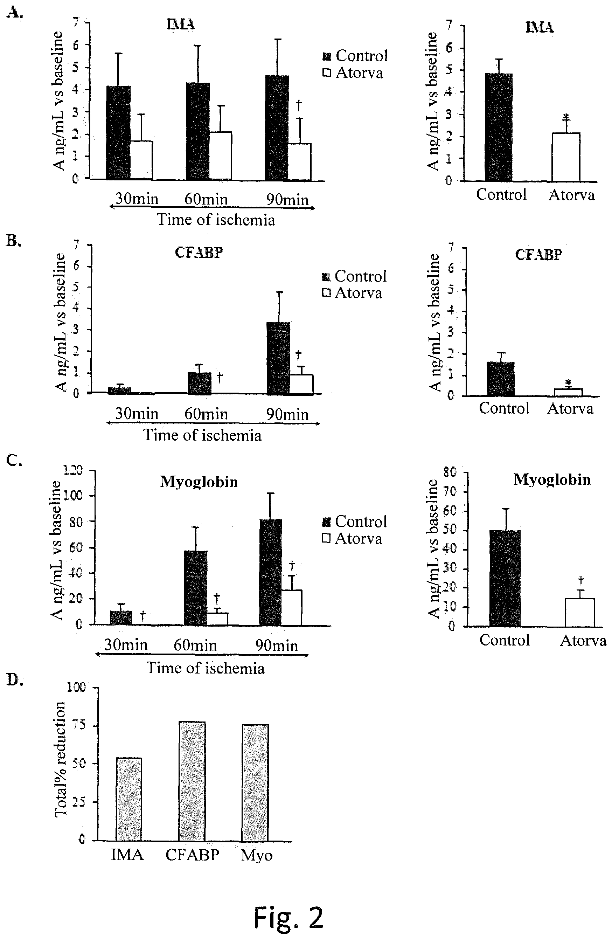

**Figure 2** shows data obtained from an experiment addressing the question whether intravenous administration of atorvastatin early after the onset of coronary occlusion protects against ischemia-related damage by studying the effects on cardiac damage-related markers over a 90 min ischemic period. **A.** IMA: Ischemic Modified Albumin; **B.** CFABP: Cardiac Fatty Acid Binding Protein; **C.** Myoglobin. **D.** Total percentage of reduction vs. control. Results are expressed as increase vs baseline levels. *p<0.05 vs Control and over time; ^{†}p<0,05 vs Control. N=7 animals/group.

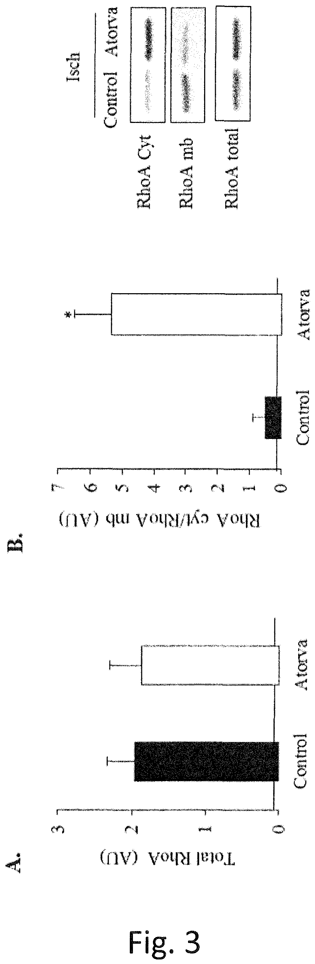

**Figure 3** is data showing that atorvastatin inhibits RhoA activation (translocation to the cell membrane) in the ischemic myocardium. **A.** Total RhoA expression. **B.** RhoA inhibition expressed as ratio between RhoA detected in the cytoplasm (cyt; inactive form) vs. RhoA detected in the membrane (mb; active form). *P<0.05 vs control; isch: ischemic myocardium.

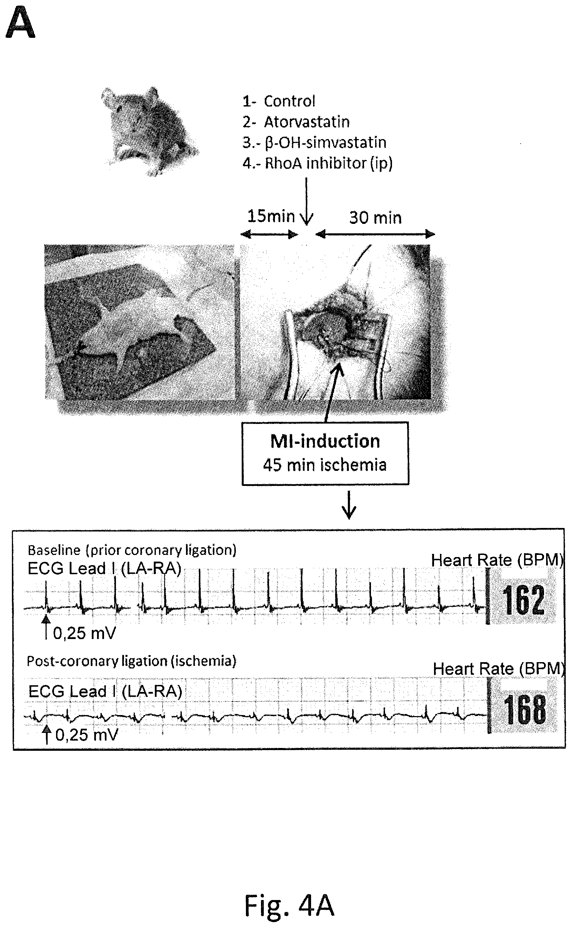

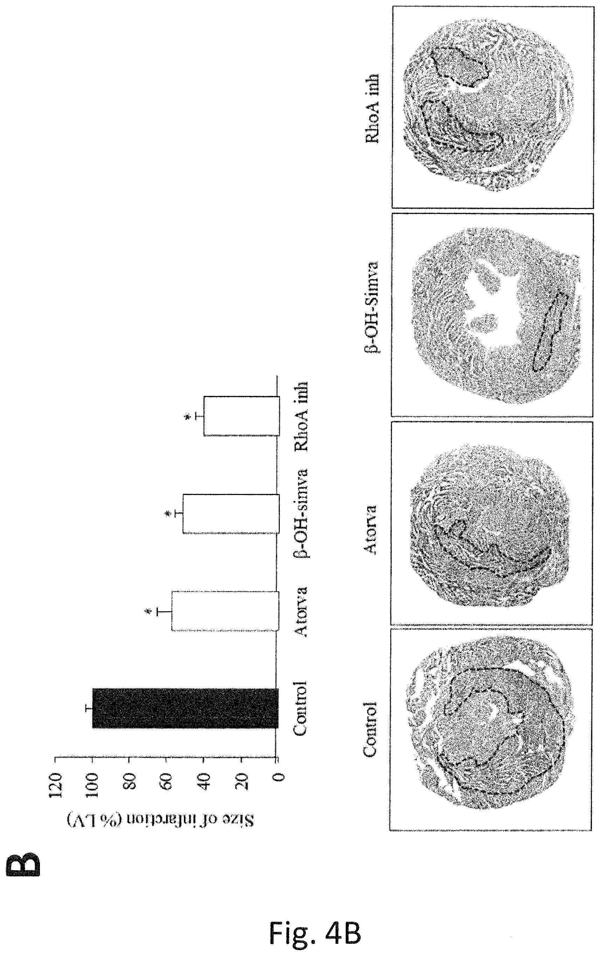

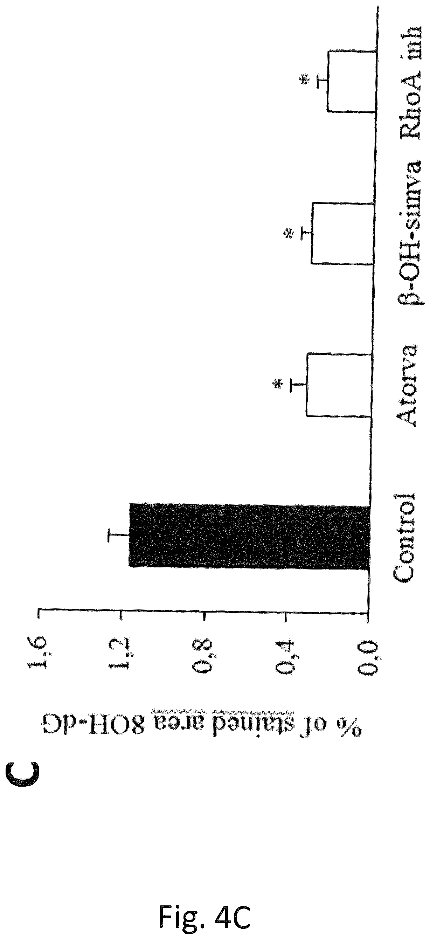

**Figure 4** is data from a mouse model of coronary ligation showing the cardioprotective effects exerted by different types of statins (atorvastatin and β-OH-simvastatin) and a specific RhoA inhibitor (CCG1423). **(A)** Coronary artery ligation and ischemia induction assessed by EGC. **(B)** Histological analysis of infarct size. **(C)** Histological analysis of oxidative damage by 8-hydroxy guanosine staining (marker of DNA oxidative damage). **p<0.05 vs Control. LV: left ventricle; Atorva: atorvastatin; β-OH-simva: beta-hydroxy acid form of simvastatin; RhoA inh: RhoA inhibitor.*

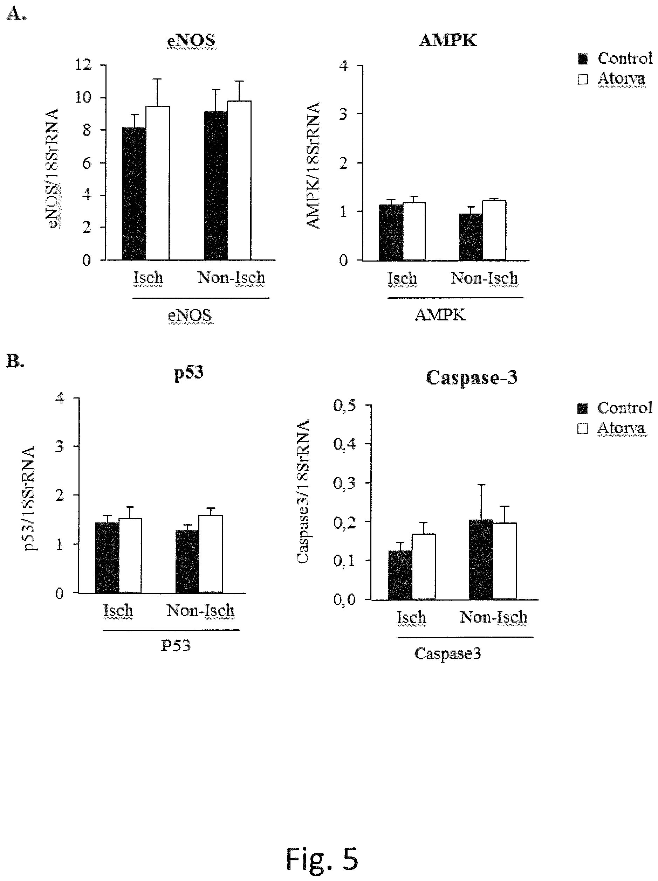

**Figure 5** shows the effect of atorvastatin on **(A)** myocardial transcript levels of the cardioprotective molecules AMPK and eNOS (endothelial nitric oxide synthase) and **(B)** apoptosis-related markers p53 and Caspase-3. Isch: ischemic myocardium; Non-Isch: non-ischemic/remote myocardium.

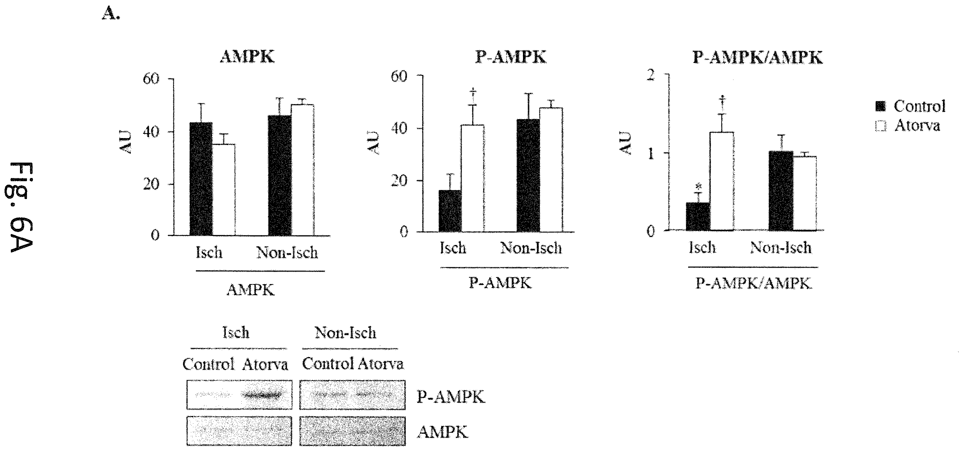

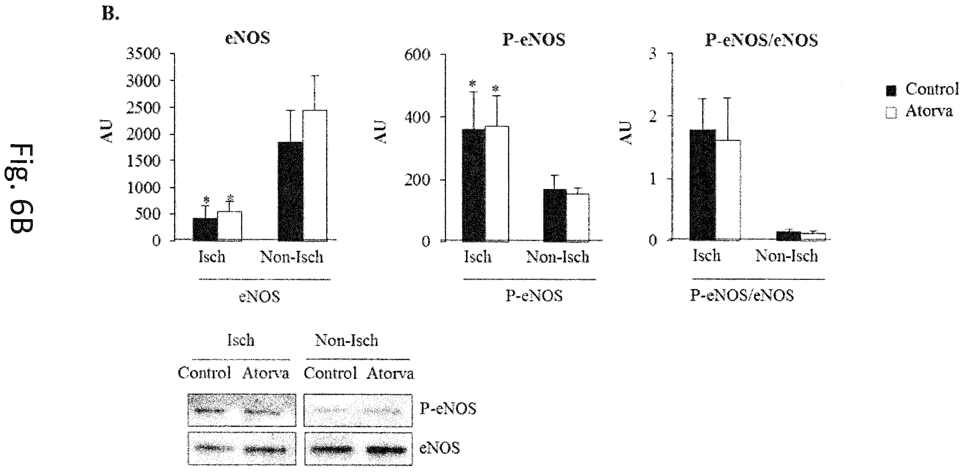

**Figure 6** shows the effect of atorvastatin on protein expression/activation and as can be seen in the figure, atorvastatin activates (phosphorylates) AMPK **(A)** whereas atorvastatin does not further enhance ischemia-induced eNOS activation **(B).** *p<0.05 vs non-isch; ^{†}p<0.05 vs Control; Atorva: atorvastatin. Isch: ischemic myocardium; Non-Isch: non-ischemic/remote myocardium.

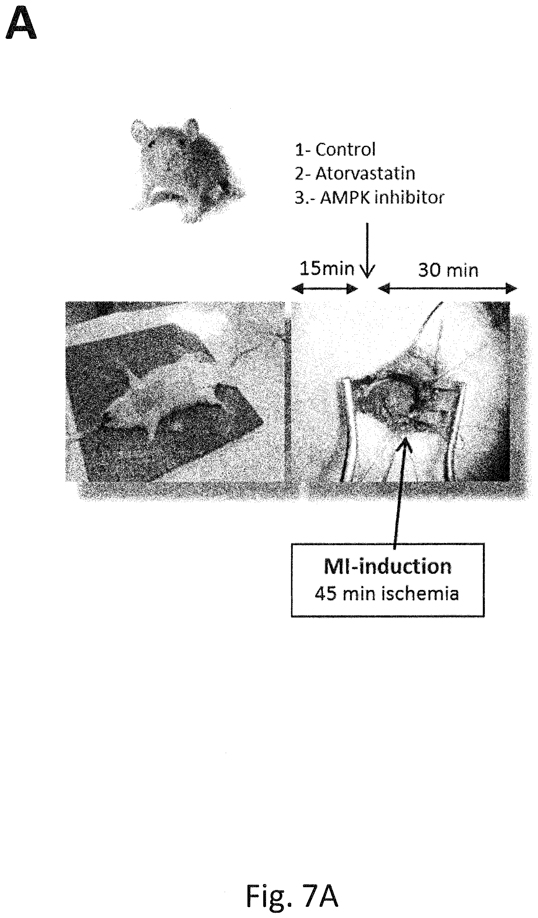

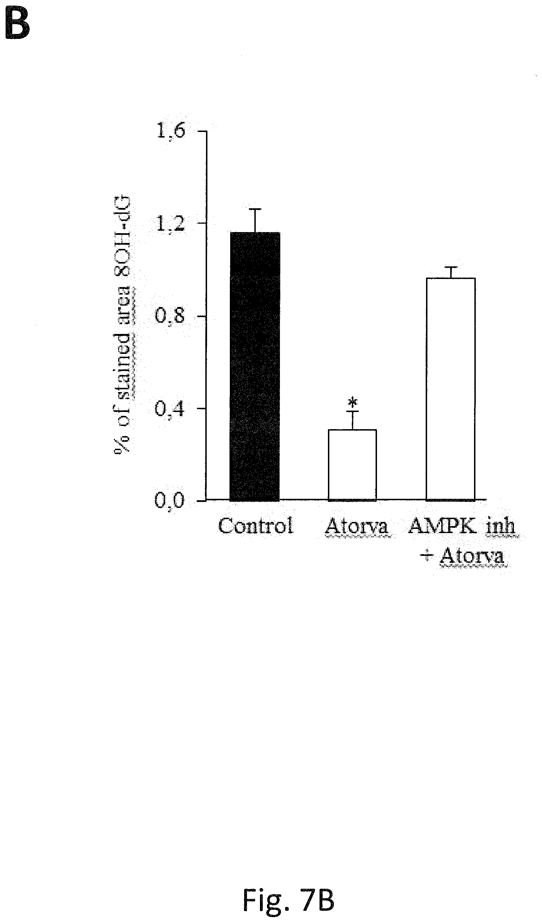

**Figure 7** is data from an experiment showing that administration of an AMPK-inhibitor (i.e, C-compound) before atorvastatin administration abolishes atorvastatin-related cardioprotective effects. **(A)** Effects on infarct size. **(B)** Effects on oxidative damage. *p<0.05 vs Control and AMPK inh+atorvastatin; IS: infarct size; atorva: atorvastatin.

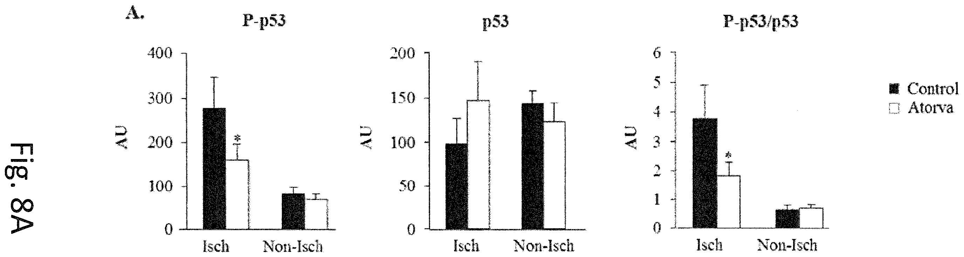

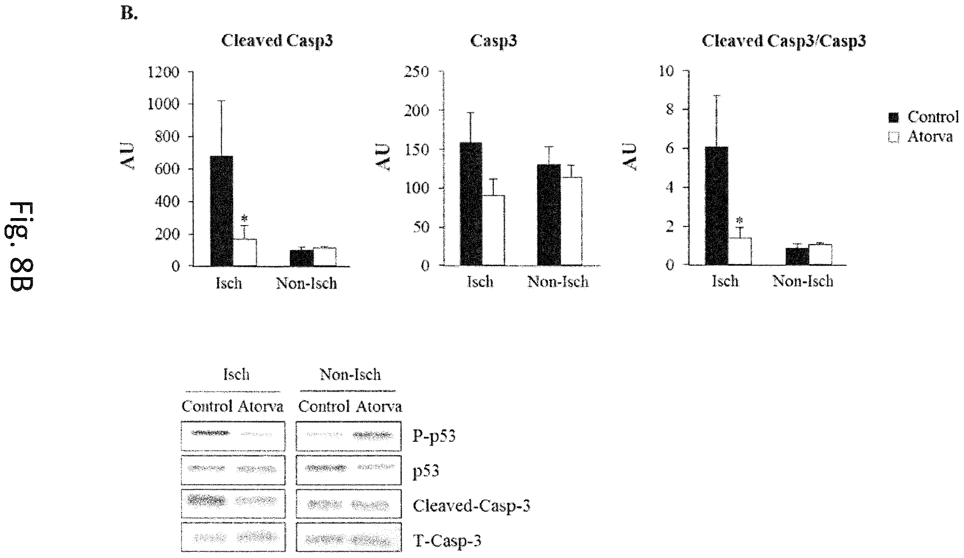

**Figure 8** shows data that intravenous administration of atorvastatin reduces apoptosis execution assessed by activation of p53 (P-p53; Figure 8A) and caspase3 (cleaved caspase3; Figure 8B) in the ischemic myocardium. **p<0.05 vs Control-Isch group; n*=*7 animals*/*group. Atorva: atorvastatin; Isch: ischemic myocardium; Non-Isch: non-ischemic myocardium.Casp3: caspase3; T-Casp3: total caspase3.*

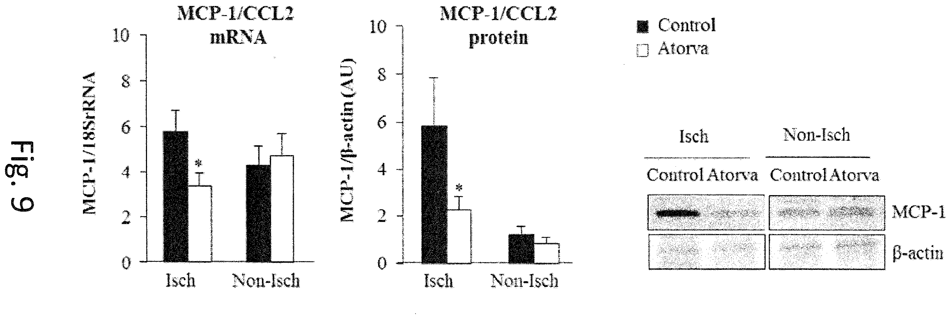

**Figure 9** shows that intravenous administration of atorvastatin attenuates myocardial MCP-1/CCL2 expression.*p<0.05 vs Control-Isch; n=7 animals/group. Atorva: atorvastatin; Isch: ischemic myocardium; Non-isch: non-ischemic myocardium.

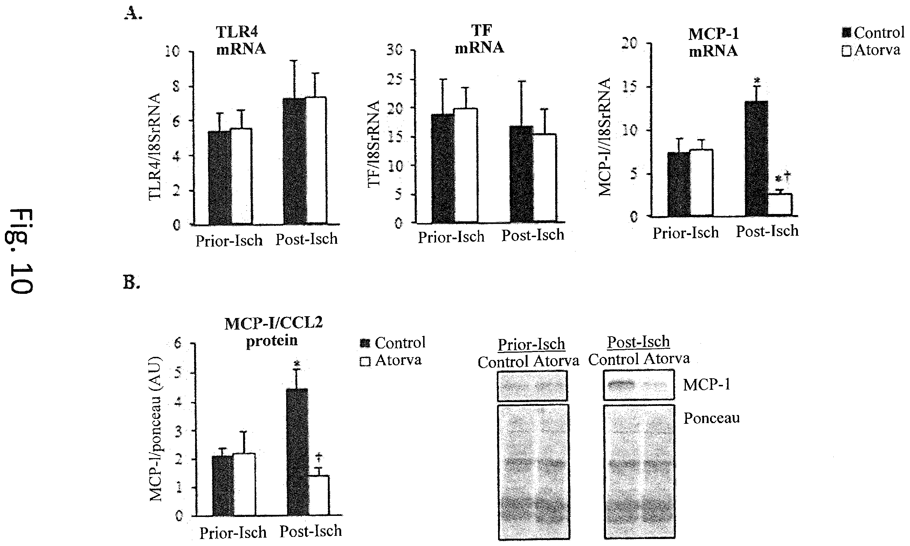

**Figure 10** shows data providing evidence that the anti-inflammatory effects of atorvastatin expand to the systemic circulation by preventing ischemia-related MCP-1/CCL2 induction in PBMC. *p<0.05 vs prior-isch; ^{†}p<0.05 vs Control post -isch; Atorva: atorvastatin-treated animals; Isch: ischemia.

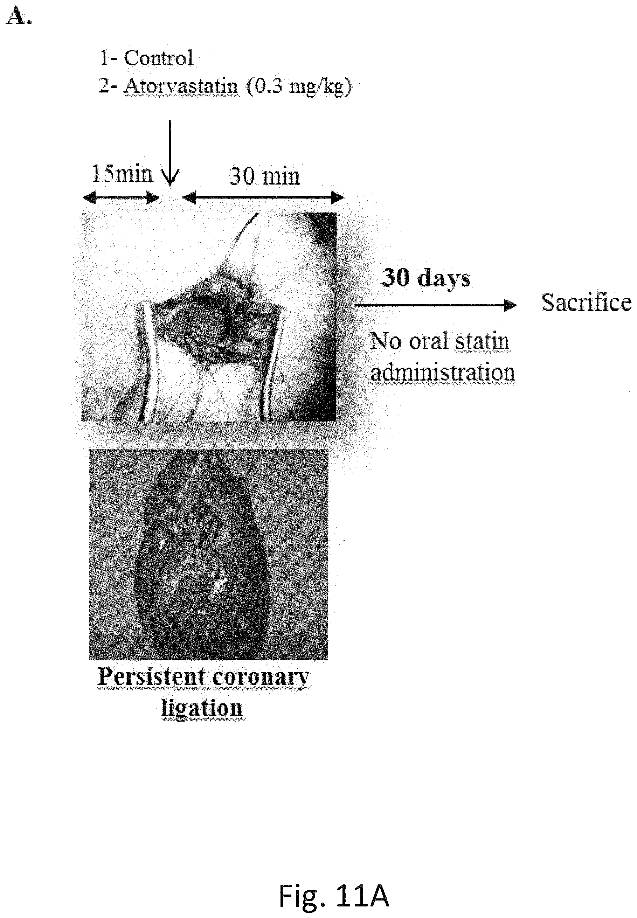

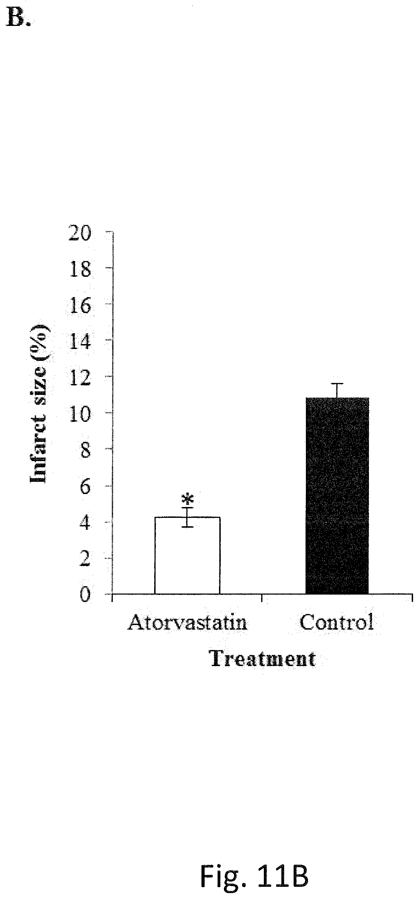

**Figure 11** shows data from a rat model of myocardial infarction by persistent coronary ligation (Figure 11A) addressing the question whether a single intravenous dose of atorvastatin early after ischemia reduces myocardial scar formation assessed 30 days after inducing ischemia (Figure 11B).

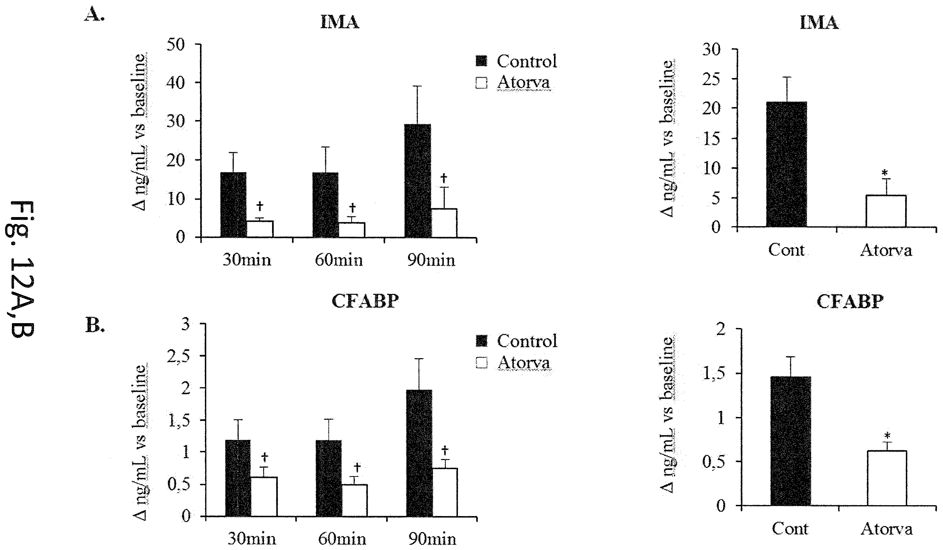

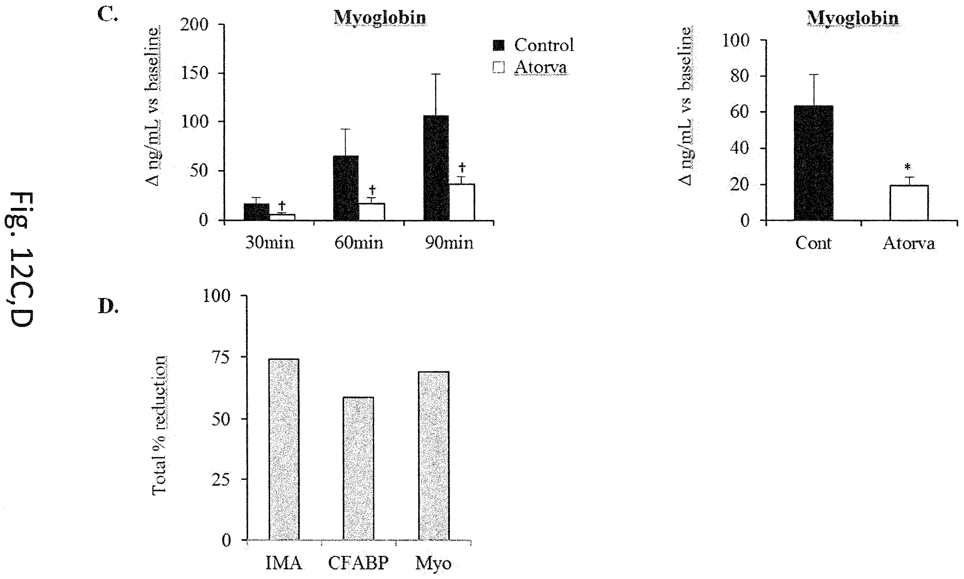

**Figure 12** shows data obtained from an experiment in hypercholesterolemic pigs addressing the question whether intravenous administration of atorvastatin early after the onset of coronary occlusion protects against ischemic damage even in the presence of hypercholesterolemia by studying the effects on cardiac damage-related markers over a 90 min ischemic period. **(A)** IMA: Ischemic Modified Albumin; **(B)** CFABP: Cardiac Fatty Acid Binding Protein; **(C)** Myoglobin; **(D)** Quantification of reduction vs. Control. *p<0.05 vs Control and over time; ^{†} p<0.05 vs control.

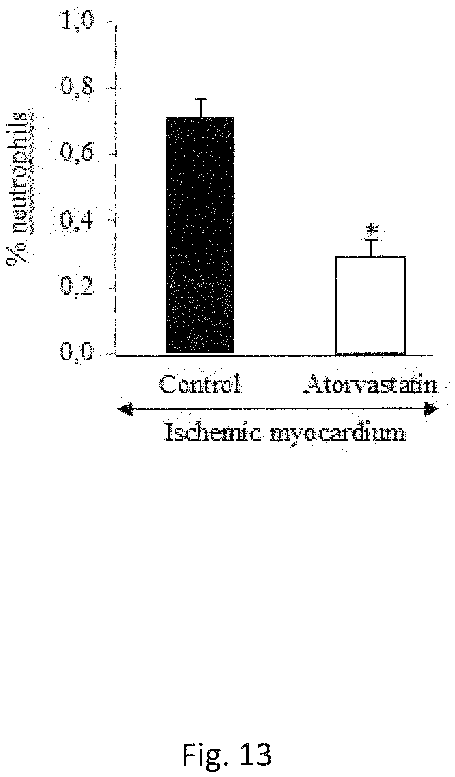

**Figure 13** shows that intravenous administration of atorvastatin to hypercholesterolemic pigs early after the onset of coronary occlusion (ischemia) reduces neutrophil infiltration in the ischemic myocardium. *p<0.05 vs Control group.

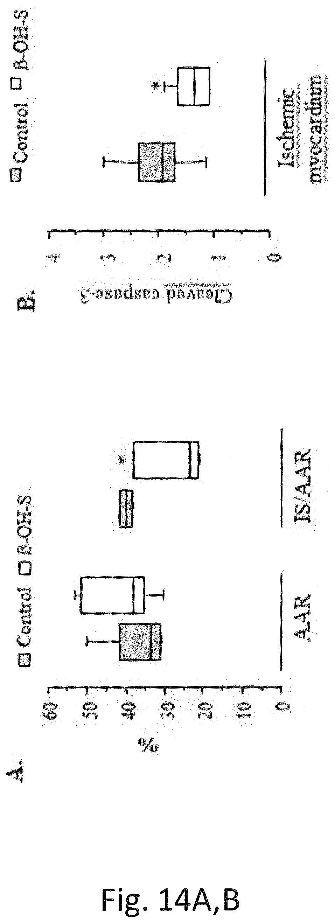

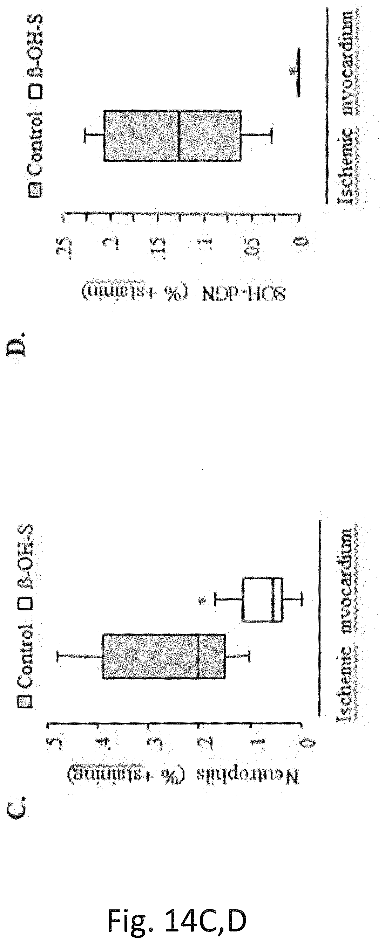

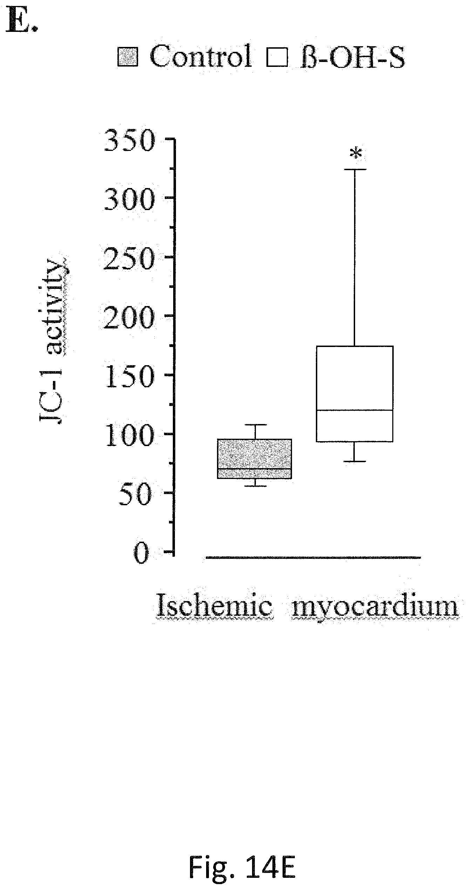

**Figure 14** shows in a pig model of ischemia and reperfusion that intravenous administration of a statin (β-OH-Simvastatin) during ischemia protects the heart against reperfusion damage associated to ischemic damage leading to an overall reduction of infarct size **(A),** and, within the ischemic myocardium, a decrease in caspase-3 activation **(B),** neutrophils infiltration **(C),** oxidative stress-related damage **(D),** and a higher preservation in mitochondrial membrane potential (JC-1 activity; **E**).

### DETAILED DESCRIPTION OF THE INVENTION

### 1. Definitions

In order to facilitate understanding of the present patent application, the meaning of some terms and expressions in the context of the present disclosure will be explained below.

The term "ischemia", as used herein, relates to a condition that may occur in any organ or tissue that is suffering a lack of oxygen supply and/or metabolites supply. This occurs when there is an imbalance between oxygen supply and demand, due to inadequate perfusion (i.e. blood supply). Insufficient oxygen supply may be caused by the formation of a thrombus, the presence of stenotic atherosclerotic lesions, restenosis, anemia, stroke, clotted arteries, vasoconstriction and/or endothelial dysfunction of the microvasculature (Takotsubo syndrome).

The term "ischemia damage" and "ischemic damage" are used synonymously herein. These terms relate to organ or tissue damage caused due to insufficient blood supply to the organ or tissue during a period of ischemia before the onset of reperfusion (i.e. ischemia damage is the damage caused by ischemia during the time between the onset of ischemia and the onset of reperfusion). The typical and pathognomonic manifestation of ischemic damage is that the ischemic zone becomes pale. In contrast, within reperfusion the non-necrotic ischemic tissue recovers its physiological color.

Biochemically, ischemic damage is characterized by changes in pH (acidification), changes in ATP concentration, increased susceptibility of platelets to get activated, enhanced inflammatory reactivity both locally in the ischemic tissue and systemically in the blood (leukocytes, particularly PBMCs).

The ischemia damage can for example be caused by atherosclerosis, thrombosis, thromboembolism, lipid-embolism, bleeding, stent, surgery, angioplasty, bypass during surgery, organ transplantation, total ischemia, myocardial infarction, vasoconstriction, microvascular dysfunction and/or a combination of two or more of these. Preferably, the ischemia is due to atherosclerotic plaque rupture with superimposition of a thrombus (i.e., myocardial infarction).

Said ischemia damage may involve myocyte cell death (preferably through necrosis and/or apoptosis, more preferably through necrosis), damage caused by intracellular pH acidification due to ischemia and/or damage caused by an inflammatory response that is triggered by ischemia and further amplified during reperfusion. Preferably, said ischemia damage consists of damage caused by intracellular pH acidification due to ischemia. For example, said intracellular pH acidification may be an acidosis below pH 6.7, which causes a damaging effect on the myocardium (e.g., contractile failure). Preferably, said intracellular pH acidification due to ischemia has a damaging effect on the myocardium causing contractile failure.

During ischemia, anaerobic metabolism prevails, which produces a decrease in cell pH. To buffer this accumulation of hydrogen ions, the Na⁺/H⁺ exchanger excretes excess hydrogen ions, which produces a large influx of sodium ions (see e.g. Figure 6.1 of the publication Kalogeris et al., Int Rev Cell Mol Biol. 2012; 298: 229-317, which summarizes major pathologic events contributing to ischemic and reperfusion components of tissue injury). Ischemia also depletes cellular ATP which inactivates ATPases (e.g., Na⁺/K⁺ ATPase), reduces active Ca²⁺ efflux, and limits the reuptake of calcium by the endoplasmic reticulum (ER), thereby producing calcium overload in the cell. These changes are accompanied by opening of the mitochondrial permeability transition (MPT) pore, which dissipates mitochondrial membrane potential and further impairs ATP production. In the heart, these cellular changes are accompanied by activation of intracellular proteases (e.g., calpains) which damage myofibrils and produce hypercontracture and contracture band necrosis. These alterations and thus the degree of tissue injury vary in extent with the magnitude of the decrease in the blood supply and with the duration of the ischemic period (Kalogeris et al., Int Rev Cell Mol Biol. 2012; 298: 229-317).

Ischemia damage may involve the symptoms chest discomfort, shortness of breath, discomfort in other areas of the upper body, feeling sick, and/or anxiety.

The term "reperfusion", as used herein, relates to the restoration of blood flow to the ischemic tissue. Despite the unequivocal benefit of reperfusion of blood to an ischemic tissue, it is known that reperfusion itself can elicit a cascade of adverse reactions that paradoxically injure the tissue.

During acute myocardial ischemia the intracellular pH decreases to less than 7.0, whereas at reperfusion, physiological pH is rapidly restored by the washout of lactate and the activation of the Na⁺-H⁺ exchanger as well as the Na⁺-HCO⁻ symporter. This abrupt pH shift contributes to the cardiomyocyte death of lethal myocardial reperfusion injury by permitting MPTP (mitochondrial permeability transition pore) opening and cardiomyocyte rigor hypercontracture in the first few minutes of reperfusion. Reperfusion of ischemic animal hearts with an acidic buffer can reduce MI (myocardial infarction) size (Hausenloy DJ and Yellon DM, J Clin Invest. 2013 Jan 2; 123(1): 92-100).

The term "reperfusion damage", as used herein, relates to organ or tissue damage caused when blood supply returns to the organ or tissue after a period of ischemia. Thus, reperfusion damage is the damage caused during the time between at the onset of reperfusion and the end of reperfusion (typically the major part of this damage will be caused during the first minutes of reperfusion). The mechanisms underlying reperfusion injury are complex, multifactorial and involve (1) generation of reactive oxygen species (ROS) that is fueled by reintroduction of molecular oxygen when the blood flow is reestablished, (2) calcium overload, (3) opening of the MPT pore, (4) endothelial dysfunction, (5) appearance of a prothrombogenic phenotype, and (6) pronounced inflammatory responses (Yellon and Hausenloy, New England Journal of Medicine (2007), Sep. 13; 357(11):1121-35). The absence of oxygen and nutrients from blood during the ischemic period creates a condition in which the restoration of circulation results in inflammation and oxidative damage through the induction of oxidative stress rather than restoration of normal function. Oxidative stress associated with reperfusion may cause damage to the affected tissues or organs. Reperfusion damage is characterized biochemically by a depletion of oxygen during an ischemic event followed by reoxygenation and the concomitant generation of reactive oxygen species during reperfusion. The injury that occurs with reperfusion is the result of the interaction between the substances that accumulate during ischemia and those that are delivered on reperfusion. The cornerstone of these events is oxidative stress, defined as the imbalance between oxygen radicals and the endogenous scavenging system. The result is cell injury and death, which is initially localized, but eventually becomes systemic if the inflammatory reaction is unchecked.

Reperfusion damage, mainly characterized by a burst of oxygen and inflammatory response, and consequent tissue damage may occur after revascularization of the infarcted (ischemic) tissue (e.g. heart). It is associated with the impairment in mitochondrial membrane potential, further apoptosis execution, reperfusion-related arrhythmias, cardiac stunning and overall increase in the size of infarction induced by ischemia. Consequently, the final infarct size (tissue damage) depends on both the ischemia damage (the tissue damage caused during the ischemic period per se), and to a lesser extent, the tissue damage caused by reperfusion.

Reperfusion damage can be caused for example by a mechanical event (e.g., restoration of blood flow following a myocardial infarction), or by one or more surgical procedures or other therapeutic interventions that restore blood flow to a tissue or organ that has been subjected to a diminished supply of blood (e.g. during transplantation). Such surgical procedures include, for example, coronary artery bypass graft surgery, coronary angioplasty, organ transplant surgery and the like (e.g., cardiopulmonary bypass surgery). In a particular embodiment, reperfusion damage is due to the treatment of ischemic process due to atherosclerotic plaque rupture/erosion with the superimposition of a thrombus (myocardial infarction), thromboembolism, lipid-embolism, bleeding, stent, surgery, angioplasty, end of bypass during surgery, organ transplantation, total ischemia, vasoconstriction, or microvascular dysfunction or combinations thereof.

Reperfusion damage may involve oxidative damage, and damage due to an inflammatory reaction that, although weak, is triggered during ischemia and becomes pronounced at reperfusion, and/or myocyte cell death. Preferably, reperfusion damage involves oxidative damage, damage due to an inflammatory reaction and myocyte cell death. More preferably, reperfusion damage involves oxidative damage, damage due to an inflammatory reaction and myocyte cell death, but not due to intracellular pH acidification.

Reperfusion damage may involve the symptoms palpitations, acute respiratory distress, fatigue, and/or edemas.

The term "ischemia-related damage", as used herein, refers to the sum of the damage induced during the ischemia (the ischemia damage) and the damage produced during reperfusion that is associated to the ischemic damage (the reperfusion damage). Thus, ischemia-related damage is the damage caused during the time between the onset of ischemia and the end of reperfusion. In the most preferred embodiments of the present disclosure, "ischemia-related damage" is limited to ischemia damage, i.e. the damage caused by ischemia during the time between the onset of ischemia and the onset of reperfusion (i.e. ischemia-related damage is the damage caused during the ischemic period after the onset of ischemia, but prior to reperfusion and thus includes the "ischemia damage", but does not include the "reperfusion damage").

In a preferred embodiment, said ischemia-related damage consist of at least one damage selected from the group consisting of myocyte cell death (preferably through necrosis and/or apoptosis, more preferably through necrosis), damage caused by intracellular pH acidification due to ischemia and/or damage caused by an inflammatory response due to ischemia that is, preferably, triggered by ischemia and further amplified during reperfusion. In more preferred embodiments, said ischemia-related damage consists of myocyte cell death (preferably through necrosis and/or apoptosis, more preferably through necrosis), damage caused by intracellular pH acidification due to ischemia and damage caused by an inflammatory response due to ischemia that is, preferably, triggered by ischemia and further amplified during reperfusion. In even more preferred embodiments, said ischemia-related damage consists of damage caused by intracellular pH acidification due to ischemia.

Myocyte cell death can be detected by molecular analysis of apoptotic/necrotic markers. pH acidification can be assessed by NMR spectrometry. An inflammation can be detected by serum assessment of inflammatory markers (e.g., IL6, TNFalpha).

In a particularly preferred embodiment, said ischemia-related damage consists of damage to myofibrils caused by intracellular proteases (such as calpains) which, preferably, causes hypercontracture and/or contracture band necrosis. In the most preferred embodiment, said ischemia-related damage consists of damage to myofibrils caused by intracellular proteases (preferably calpains) which causes hypercontracture and/or contracture band necrosis.

Myocyte ultrastructural changes can be assessed by electron microscopy of myocardial biopsies.

The term "prevention", as used herein e.g. in the context of prevention of ischemia-related damage, relates to the capacity to prevent, minimize or hinder the onset or development of a disease or condition before its onset.

The term "reduction", as used herein in the context of reduction of ischemia-related damage, relates to the capacity to limit and/or reduce a disease or condition that has already developed.

The term "administration" or the statement that a compound is "administered" to a subject, as used herein, relates to the delivery of a pharmaceutical drug to a subject. The suitable route for administration of the statin for use according to the present disclosure is by intravenous administration to the subject in need thereof.

If the application states that a compound is to be administered "as early as possible after the onset of ischemia", this preferably means that the compound is to be administered not more than 60 minutes after the onset of ischemia. In a preferred embodiment, it means that the compound is to be administered not more than 45 minutes after the onset of ischemia. In a more preferred embodiment, it means that the compound is to be administered not more than 30 minutes after the onset of ischemia. In a more preferred embodiment, it means that the compound is to be administered not more than 20 minutes after the onset of ischemia. In a even more preferred embodiment, it means that the compound is to be administered not more than 15 minutes after the onset of ischemia.

In some embodiments, the compound is to be administered at least 15 minutes prior to reperfusion, preferably at least 20 minutes prior to reperfusion, even more preferably at least 30 minutes prior to reperfusion, most preferably at least 45 minutes prior to reperfusion.

In some embodiments, the statement that the compound is to be administered "as early as possible after the onset of ischemia" means that the compound is to be administered not more than 60 minutes after the onset of ischemia, and the compound is to be administered at least 15 minutes prior to reperfusion. In some embodiments, the statement that the compound is to be administered "as early as possible after the onset of ischemia" means that the compound is to be administered not more than 60 minutes after the onset of ischemia, and the compound is to be administered at least 20 minutes prior to reperfusion. In some embodiments, the statement that the compound is to be administered "as early as possible after the onset of ischemia" means that the compound is to be administered not more than 60 minutes after the onset of ischemia, and the compound is to be administered at least 30 minutes prior to reperfusion. In some embodiments, the statement that the compound is to be administered "as early as possible after the onset of ischemia" means that the compound is to be administered not more than 60 minutes after the onset of ischemia, and the compound is to be administered at least 45 minutes prior to reperfusion.

In some embodiments, the statement that the compound is to be administered "as early as possible after the onset of ischemia" means that the compound is to be administered not more than 45 minutes after the onset of ischemia, and the compound is to be administered at least 15 minutes prior to reperfusion. In some embodiments, the statement that the compound is to be administered "as early as possible after the onset of ischemia" means that the compound is to be administered not more than 45 minutes after the onset of ischemia, and the compound is to be administered at least 20 minutes prior to reperfusion. In some embodiments, the statement that the compound is to be administered "as early as possible after the onset of ischemia" means that the compound is to be administered not more than 45 minutes after the onset of ischemia, and the compound is to be administered at least 30 minutes prior to reperfusion. In some embodiments, the statement that the compound is to be administered "as early as possible after the onset of ischemia" means that the compound is to be administered not more than 45 minutes after the onset of ischemia, and the compound is to be administered at least 45 minutes prior to reperfusion.

In some embodiments, the statement that the compound is to be administered "as early as possible after the onset of ischemia" means that the compound is to be administered not more than 30 minutes after the onset of ischemia, and the compound is to be administered at least 15 minutes prior to reperfusion. In some embodiments, the statement that the compound is to be administered "as early as possible after the onset of ischemia" means that the compound is to be administered not more than 30 minutes after the onset of ischemia, and the compound is to be administered at least 20 minutes prior to reperfusion. In some embodiments, the statement that the compound is to be administered "as early as possible after the onset of ischemia" means that the compound is to be administered not more than 30 minutes after the onset of ischemia, and the compound is to be administered at least 30 minutes prior to reperfusion. In some embodiments, the statement that the compound is to be administered "as early as possible after the onset of ischemia" means that the compound is to be administered not more than 30 minutes after the onset of ischemia, and the compound is to be administered at least 45 minutes prior to reperfusion.

In some embodiments, the statement that the compound is to be administered "as early as possible after the onset of ischemia" means that the compound is to be administered not more than 20 minutes after the onset of ischemia, and the compound is to be administered at least 15 minutes prior to reperfusion. In some embodiments, the statement that the compound is to be administered "as early as possible after the onset of ischemia" means that the compound is to be administered not more than 20 minutes after the onset of ischemia, and the compound is to be administered at least 20 minutes prior to reperfusion. In some embodiments, the statement that the compound is to be administered "as early as possible after the onset of ischemia" means that the compound is to be administered not more than 20 minutes after the onset of ischemia, and the compound is to be administered at least 30 minutes prior to reperfusion. In some embodiments, the statement that the compound is to be administered "as early as possible after the onset of ischemia" means that the compound is to be administered not more than 20 minutes after the onset of ischemia, and the compound is to be administered at least 45 minutes prior to reperfusion.

In some embodiments, the statement that the compound is to be administered "as early as possible after the onset of ischemia" means that the compound is to be administered not more than 15 minutes after the onset of ischemia, and the compound is to be administered at least 15 minutes prior to reperfusion. In some embodiments, the statement that the compound is to be administered "as early as possible after the onset of ischemia" means that the compound is to be administered not more than 15 minutes after the onset of ischemia, and the compound is to be administered at least 20 minutes prior to reperfusion. In some embodiments, the statement that the compound is to be administered "as early as possible after the onset of ischemia" means that the compound is to be administered not more than 15 minutes after the onset of ischemia, and the compound is to be administered at least 30 minutes prior to reperfusion. In some embodiments, the statement that the compound is to be administered "as early as possible after the onset of ischemia" means that the compound is to be administered not more than 15 minutes after the onset of ischemia, and the compound is to be administered at least 45 minutes prior to reperfusion.

The term "statin", as used herein, relates to a 3-hydroxy-3-methyl-glutaryl-CoA reductase inhibitor, i.e. an inhibitor of the HMG-CoA reductase enzyme, which catalyzes the limiting step of cholesterol biosynthesis, e.g., the conversion of HMG-CoA to mevalonate. The term includes any natural, synthetic or semisynthetic statin. Inhibition of HMG-CoA reductase by statins decreases intracellular cholesterol biosynthesis in liver and extrahepatic tissues.

Recently, the scope of statin therapy has expanded with the emergence of evidence suggesting that due to pleiotropic effects of statins that are not directly associated with their regulation of cholesterol levels, they may prove to be beneficial for treating a number of diseases. The pleiotropic effects associated with statins that may impact disease pathophysiology include their modulation of immune responses, their enhancement of anti-inflammatory processes, and their alterations of signaling pathways that involve cholesterol intermediates. To date, the multitude of diseases linked to the pleiotropic effects of statins include multiple sclerosis (MS), inflammatory bowel diseases (IBDs), rheumatoid arthritis (RA), systemic lupus erythematosus (SLE), chronic obstructive pulmonary disease (COPD), cancer, stroke, Parkinson's and Alzheimer's diseases, bacterial infections, HIV, and programmed percutaneous coronary intervention. These utilities have been observed with a chronic or sub-chronic administration of statins (e.g. in patients undergoing percutaneous coronary intervention, reduction of procedural myocardial injury after 7-day pretreatment with atorvastatin was paralleled by concomitant attenuation of post-procedural increase of intercellular cell adhesionmolecule-1 (ICAM-1) and E-selectin levels).

Some statins can be in the closed form (lactone) or in the open form (hydroxy acid). Hydroxy acids (open form) can be prepared from the corresponding lactones by conventional hydrolysis, for example, with sodium hydroxide in methanol, sodium hydroxide in tetrahydrofuran- water and the like. In the open form (hydroxy acid), the statins react to form salts with pharmaceutically acceptable metal and amine cations formed from organic or inorganic bases. The pharmaceutically acceptable salts of the statins can differ from the corresponding free acids in some physical characteristics such as solubility and melting point. The free open form (hydroxy acid) of the statins can be regenerated from the salt form, if desired, by contacting the salt with a diluted aqueous solution of an acid such as hydrochloric acid and the like. The closed form (lactone) of the statins can be regenerated by dissolving the open form (hydroxy acid) in an inert solvent such as, for example, toluene, benzene, ethyl acetate and the like, at temperatures comprised between approximately 0°C and approximately the boiling point of the solvent, typically (although not necessarily) with simultaneous separation of the resulting water and catalysis with strong acids, e.g., hydrochloric acid and the like.

As a skilled person will be aware, the reaction of HMG-CoA reductase is also a step of the pathway leading to formation of geranylgeranyl-PP. Geranylgeranyl-PP is required for geranylgeranylation of RhoA, a step in the activation of RhoA. Hence, statins typically also have an inhibitory activity on the translocation of the RhoA protein from cytosol to cell membrane and thus on RhoA activation. The statin according to the present disclosure preferably is an inhibitor of HMG-CoA reductase and of RhoA activation.

Further statins may be obtained by forming e.g. nitroderivatives of known statins as disclosed in US 7563909 B2, or by including a lactose group (which are rapidly hydrolyzed in the presence of water) in a known statin. Moreover, further statins may also be obtained as the result of the metabolism of a known statin by a cellular enzyme, preferably resulting in an active form of the statin or statin metabolite. In a particular embodiment, the statin metabolite relates to the hydroxy acid form of a statin according to the present disclosure. Preferably, the statin metabolite is an active form of a statin, that is to say a statin form exhibiting at least 40%, at least 50%, at least 60%, at least 70%, at least 80%, at least 90% or at least 95% of the HMG-CoA reductase activity of the statin from which is obtained. As the skilled person acknowledges, statins are usually inactive in their lactone form. Generally, the active form thereof is their metabolite statin-β-hydroxy acid formed by hydrolysis by carboxyesterase activity in both plasma/liver and intestinal mucosa. In particular, the cytochrome P450 enzyme (CYP3A4) is involved in the metabolism of most statins.

The term "simvastatin", as used herein, refers to (1S,3R,7S,8S,8aR)-8-{2-[(2R,4R)-4-hydroxy-6-oxotetrahydro-2H-pyran-2-yl]ethyl}-3,7-dimethyl-1,2,3,7,8,8a hexahydronaphthalen-1-yl 2,2-dimethylbutanoate, a cholesterol-lowering compound that is widely used as a potent inhibitor of the HMG-CoA reductase enzyme to treat hypercholesterolemia. The HMG-CoA reductase enzyme catalyzes the conversion of HMG-CoA to mevalonate, which is an early and rate-limiting step in the biosynthesis of cholesterol. Chemically, simvastatin is an inactive lactone that is converted to 3',5'-dihydrodiol simvastatin in the liver by cytochrome P-450 (CYP) 3 A after oral administration.

If the present application states that a compound "inhibits RhoA activation", is a "RhoA inhibitor" or is an "agent inhibiting RhoA translocation to the cell membrane", this relates to a situation where said compound inhibits or blocks the translocation of the RhoA protein from the cytosol (inactive form of RhoA) to the cell membrane (active form of RhoA). The term "RhoA", as used herein, also known as Ras homolog gene family member A, relates to a small GTPase protein that regulates the actin cytoskeleton in the formation of stress fibers. The term "RhoA" includes any RhoA of any subject (e.g., a mammal). Human RhoA protein is encoded by the RHOA gene (GeneID: 387, mRNA nucleotide sequence of 1,926 bp with accession number NM 001664 according to the NCBI nucleotide database, as of 27 April 2014). Similar to other GTPases, RhoA contains both inactive GDP-bound and active GTP-bound states, these states alternate between the active and inactive states via the exchange of GDP to GTP. RhoA, mostly found in the cytoplasm, is primarily involved in actin organization, cell cycle maintenance, cellular development and transcriptional control. An agent inhibiting RhoA translocation to the cell membrane may also have, in addition to its own activity on the translocation of the RhoA protein from cytosol to cell membrane.

The term "subject" or "individual" or "animal" or "patient" or "mammal", is meant any subject, particularly a mammalian subject, for whom therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the present disclosure, the subject is a mammal. In the most preferred embodiment of the present disclosure, the subject is a human.

The term "treatment", as used herein, refers to both therapeutic measures and prophylactic or preventive measures, wherein the object is to prevent or slow down (lessen) an undesired physiological change or disorder, such as ischemic damage. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

### 2. Use of a statin in the prevention and/or reduction of ischemia-related damage

In an aspect, the present disclosure relates to a compound for use in the prevention and/or reduction of ischemia-related damage in a subject, wherein said compound is a statin (i.e. a 3-hydroxy-3-methyl-glutaryl-CoA reductase inhibitor), wherein said compound is to be administered to said subject by intravenous administration after the onset of ischemia, but prior to reperfusion, wherein the compound is to be administered as soon as possible after the onset of ischemia.

Thus, according to the present disclosure, the compound for use in the prevention and/or reduction of ischemia-related damage in a subject is a statin.

Statins are compounds that inhibit the HMG-CoA reductase enzyme, which is the enzyme catalyzing the conversion of HMG-CoA to mevalonate. Assays to determine the HMG-CoA reductase activity of a compound are known by the skilled person and include, without limitation, those described by Liu (Liu L et al. 2003 J Pharm Biomed Anal 32(1): 107-123), Fang (Fang W et al. 2002 J Clin Lab Anal 16(5): 209-215), or Mozzicafreddo (Mozzicafreddo M et al. 2010 J Lipid Res 51(8): 2460-2463), as well as by means of kits commercially available by, without limitation, Sigma- Aldrich (Catalog No. CS1090). Statins exert mainly a function as inhibitors of the enzyme HMG-CoA reductase. As it is well-known, a statin will typically also have an inhibitory activity on the translocation of the RhoA protein from the cytosol to cell membrane.

A first group of statins comprises statins with a substituted decalin-ring structure and includes lovastatin, pravastatin and simvastatin. Lovastatin, pravastatin and simvastatin are inactive lactones which must be metabolized to their active hydroxy-acid forms in order to inhibit the HMG-CoA reductase enzyme. Therefore, statins in the first group exert their function when converted to their hydroxy-acid form of the statin. A second group of statins comprises statins wherein the butyryl group as in the statins in the first group is replaced by a fluorophenyl group, which is responsible for additional polar interactions causing a tighter binding to the enzyme. This second group of statins includes fluvastatin, cerivastatin, atorvastatin and rosuvastatin, which are statins in an active hydroxy-acid form.

Statins are compounds that can be obtained by the skilled person by methods known in the art including, without limitation, those described in US 201 10223640 A1, US 8471045 B2, WO 2009133089 A1 and EP 1015600 A1. Statins are also commercially available from a number of suppliers including, without limitation, Pfizer and Bayer.

Examples for statins that may be used in the present disclosure are disclosed below (Table 1).

| **Statin and formula thereof** | **Further related statins** |
|---|---|
| 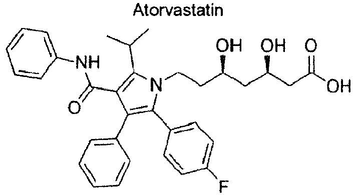 | Beta-oxidized atorvastatin, unsaturated beta-oxidized atorvastatin, beta-oxidized hydroxy atorvastatin, 4-hydroxy atorvastatin para-hydroxy atorvastatin acid, atorvastatin lactone, atorvastatin acyl glucuronide, 2-hydroxy atorvastatin ortho-hydroxy atorvastatin acid, 4-hydroxy atorvastatin acyl glucuronide, 4-hydroxy atorvastatin lactone and 2-hydroxy atorvastatin lactone. |
| 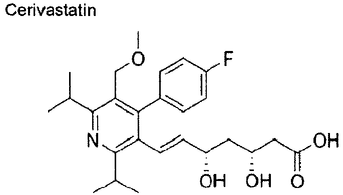 | Cerivastatin 6-O-glucuronide, cerivastatin 3-O-glucuronide, cerivastatin 4-O-glucuronide, cerivastatin lactone, |
| 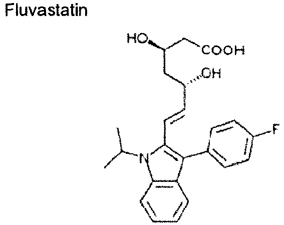 | 5-hydroxy fluvastatin, 6-hydroxy fluvastatin and N-deisopropyl fluvastatin. |
| 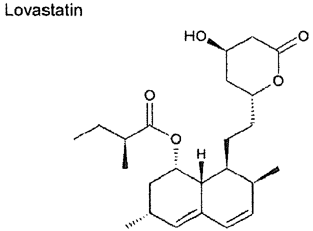 | Lovastatin acid, 3'-hydroxylovastatin, 6'-exomethylenelovastatin and 6'-beta-hydroxylovastatin and 3"β-hydroxylovastatin. |
| 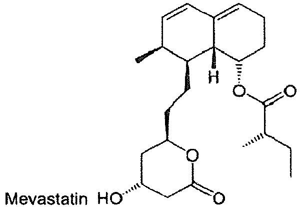 | |
| 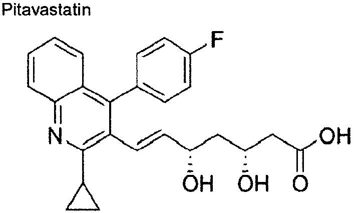 | Pitavastatin lactone, 3-keto pitavastatin, pitavastatin keto lactone, hydroxy pitavastatin lactone, 3-dehydroxy pitavastatin O-glucuronide, 5-keto pitavastatin acyl glucuronide, 5-keto pitavastatin O-glucuronide and 3-dehydroxy pitavastatin acyl glucuronide. |
| 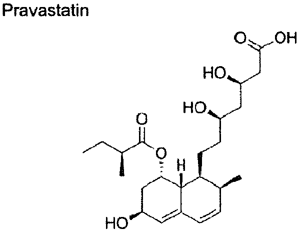 | 3 "(S)-hydroxytetranorpravastatin, 3'-alpha-5',6'-epoxyisopravastatin, 3'-alpha-7-hydroxyisopravastatin, 3'-ketopravastatin-5,6-diol, 3"(S)-hydroxypravastatin, 3'-alpha-isopravastatin, 3'-alpha-5'-beta,6'-beta-trihydroxypravastatin, 6'-epipravastatin, pravastatin glucuronide, desecylpravastatin, 4'-alpha-5'-beta-epoxypravastatin, pravastatin 4a'alpha-glutathione conjugate, pravastatin lactone, pravastatin sulfate, |
| 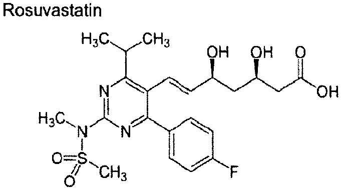 | Rosuvastatin lactone, N-demethylrosuvastatin and rosuvastatin acyl glucuronide. |
| 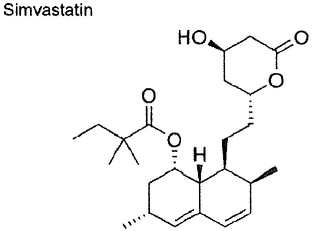 | 3',5'-dihydrodiolsimvastatin, 6'-beta-hydroxymethylsimvastatin, 6'beta-carboxysimvastatin, 6'-beta-hydroxysimvastatin, 6'-exomethylenesimvastatin, simvastatin acid terivastatin, simvastatin acid glucuronide and 3'-hydroxysimvastatin |

Preferably, the statin according to the present disclosure is selected from the group consisting of (in parenthesis examples of trade names under which the statin is sold): atorvastatin (Lipitor®, Torvast®), cerivastatin (Lipobay®, Baycol®), fluvastatin (Lescol®), lovastatin (Mecavor®, Altocor®, Altoprev®), mevastatin (Compactin®), pitavastatin (Livalo®, Pitava®), pravastatin (Pravachol®, Selektine®, Lipostat®), rosuvastatin (Crestor®), simvastatin (Zocor®, Lipex®) and the β-hydroxy acid form of simvastatin.

Even more preferably, the statin is selected from the group consisting of atorvastatin (see formula I) and the β-hydroxy acid form of simvastatin (see formula II). Most preferred is the statin atorvastatin.

The pharmacokinetic properties of the statins are orchestrated by several factors, including their active or lactone form, their lipophilic/hydrophilic rate, and their absorption and metabolism. The statin pharmacological properties, including doses administered as open acid and lactone forms, are known by the skilled person and shown, for example, in Table 1 as in Gazerro P et al. 2012 Pharmacol Rev 64(1): 102- 146*.*

The term "pharmaceutically acceptable salt" or "physiologically acceptable salt" relates to a salt formed either with a physiologically tolerated acid, that is to say salts of a compound with inorganic or organic acids which are physiologically tolerated - especially if used on mammals, preferably in human beings - or with at least one, preferably inorganic, cation which are physiologically tolerated - especially if used on mammals, preferably in human beings. Thus, a pharmaceutically acceptable salt is a molecular entity that is physiologically tolerable and that normally does not cause an allergic reaction or similar adverse reaction, such as gastric discomfort, dizziness and the like, when they are administered to a mammal, preferably a human being. Preferably, as used in this description, the term "pharmaceutically acceptable" means approved by a governmental regulatory agency or listed in the US pharmacopoeia or another generally recognized pharmacopoeia for use in mammals, and more particularly in humans. Illustrative, non- limitative examples of pharmaceutically acceptable salts of specific acids include salts of hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with NH₄. Solvates, salts and pro-drugs of the compounds of formula (I) or (II) can be prepared by methods known in the state of the art.

In a particular embodiment of the present disclosure, the statin for use according to the present disclosure in the prevention and/or reduction of ischemia-related damage in a subject is simvastatin or a statin related to simvastatin such as, for example, an oxime derivative of simvastatin showing increased aqueous solubility (Gupta A et al. 2011 Asian J Pharmac Clin Res 5(1): 50-52), 3',5'-dihydrodiol simvastatin, 6'beta-hydroxymethyl simvastatin, 6'beta-carboxy simvastatin, 6'beta-hydroxy simvastatin, 6'-exomethylene simvastatin, simvastatin acid terivastatin, simvastatin acid glucuronide and 3'-hydroxy simvastatin. In a more particular embodiment, the simvastatin derivative is a beta-hydroxy derivative of simvastatin of formula (III):

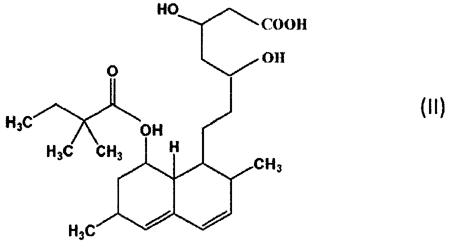

In particular, the simvastatin derivative is a beta-hydroxy derivative of simvastatin is of formula (III):

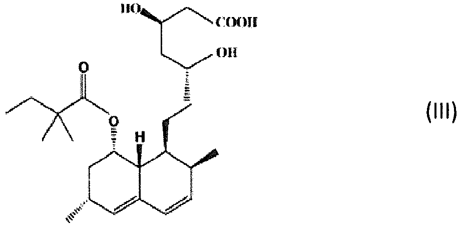

According to some embodiments, the statin inhibits RoA activation. Molecular approaches to measure cytosol-membrane translocation of RhoA, which is a sign of RhoA activation, have been described in the art by Keller (Keller J et al. 1997 FEBS Lett 403(3): 299-302). Determination of total and cytosolic-membrane fractions of RhoA according to the present disclosure has been described by some authors (*e.g.,* Vilahur G et al. 2009 Atherosclerosis 206: 95-101).

The term "therapeutically effective amount", as used herein in relation to the compound for use according to the present disclosure, relates to an amount of said compound that provides the desired effect, for example, an appreciable prevention, cure, delay, reduction of severity or amelioration of one or more symptoms derived from ischemia-related damage. The therapeutically effective amount of a compound will be generally determined by taking into consideration different features such, for example, the characteristics of the product itself and the therapeutic effect to be achieved, the particulars of the subject to be treated, the severity of the injury suffered by said subject, the chosen dosage form, etc. For this reason, the doses mentioned herein should be considered only as guides for the person skilled in the art, who must adjust the doses depending on the aforementioned variables. In an embodiment, the therapeutically effective amount of the compound is an amount that prevents, reduces, ameliorates, attenuates or eliminates one or more symptoms of ischemia damage in the treated subject.

Even though individual needs vary, determination of optimal ranges for therapeutically effective amounts of the compounds for use according to the present disclosure belongs to the common experience of those experts in the art. In general, the dosage needed to provide an effective treatment, which can be adjusted by one expert in the art, will vary depending on age, health, fitness, sex, diet, weight, degree of alteration of the receptor, frequency of treatment, nature and condition of the injury, nature and extent of impairment or illness, medical condition of the subject, route of administration, pharmacological considerations such as activity, efficacy, pharmacokinetic and toxicology profile of the particular compound used, if using a system drug delivery, and if the compound is administered as part of a combination of drugs. The amount of the compound for use according to the present disclosure that is therapeutically effective in the prevention and/or treatment of reperfusion damage in a subject can be determined by conventional clinical techniques (see, for example, The Physician's Desk Reference, Medical Economics Company, Inc., Oradell, NJ, 1995, and Drug Facts and Comparisons, Inc., St. Louis, MO, 1993).

The dosage regimen for the compound for use according to the present disclosure can vary. In a particular embodiment, the compound for use according to the present disclosure is to be administered as a perfusion to be started as soon as possible after the onset of the ischemia and to be stopped before the reperfusion. In the most preferred embodiment, the compound for use according to the present disclosure is to be administered in a single dose (i.e. by bolus administration).

Preferably, the dosage of said compound ranges from 0.1 to 1 mg/kg body weight, more preferably from 0.2 to 0.8 mg/kg body weight, most preferably from 0.3 to 0.6 mg/kg body weight.

In a particular embodiment, said compound for use is atorvastatin or the β-hydroxy acid form of simvastatin of formula (II) and the compound is to be administered to said subject in a single dose. In another embodiment, said compound for use is atorvastatin or the β-hydroxy acid form of simvastatin of formula (II) and the compound is to be administered to said subject in a single dose, wherein the dosage of the compound ranges from 0.1 to 1 mg/kg body weight, more preferably from 0.2 to 0.8 mg/kg body weight, most preferably from 0.3 to 0.6 mg/kg body weight.

The terms "pharmaceutically acceptable excipient" or "pharmaceutically acceptable carrier", refer to any compound or combination of compounds that is essentially non-toxic to the subject at the dosage and concentration employed, and is compatible with the other components of a pharmaceutical composition. Thus, an excipient is an inactive substance formulated alongside the active ingredient (i.e., the compound for use according to the present disclosure which is a statin) of a pharmaceutical composition, for the purpose of bulking-up compositions that contain said active ingredients. Bulking up allows convenient and accurate dispensation of a drug substance when producing a dosage form. Excipients also can serve various therapeutic-enhancing purposes, such as facilitating compound (drug) absorption or solubility, or other pharmacokinetic considerations. Excipients can also be useful in the manufacturing process, to aid in the handling of the active substance concerned such as by facilitating powder flowability or non-stick properties, in addition to aiding *in vitro* stability such as prevention of denaturation over the expected shelf life. The selection of appropriate excipients depends upon the route of administration and the dosage form, as well as the active ingredient and other factors. An excipient can be a non-toxic solid, semisolid or liquid filler, diluent, encapsulating material or formulation auxiliary of any conventional type. Illustrative, non-limitative, examples of excipients or carriers include water, salt (saline) solutions, alcohol, dextrose, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters, petroetrals, hydroxymethyl cellulose, polyvinylpyrrolidone and the like.

The compound for use according to the present disclosure is to be administered as soon as possible after the onset of ischemia, but prior to reperfusion. The skilled person is aware how to recognize ischemia. Myocardial ischemia, for example, is recognized by the sudden onset of characteristic symptoms: intense chest pain in precordial area, shortness of breath when you are physically active, a fast heartbeat, general malaise, fatigue, dizziness, nausea and sweating. Pain may extend to the left arm, jaw, shoulder, back, or neck. Later, myocardial ischemia can be confirmed by methods like electrocardiogram (ECG), echocardiogram.

By administering the compound as soon as possible after the onset of ischemia, it can be ensured that the ischemic period *before* administration of the compound is minimized whereas the proportion of the ischemic period *after* administration of the compound compared to the total time under ischemia (i.e. compared to the total time from the onset of ischemia to the onset of reperfusion) is maximized.

In some preferred embodiments, the statin is not administered at and/or after reperfusion.

In some preferred embodiment, the statin is not administered during reperfusion.

The statin is to be administered by intravenous administration. This can be achieved by standard procedures known to the person of skill in the art. The present inventors have observed that for the purposes of the present disclosure it is important that the statin reaches the ischemic tissue as soon as possible after the onset of ischemia, and intravenous administration has turned out to be advantageous in this regard, compared e.g. to oral administration.

In a particular embodiment, the pharmaceutical composition containing the compound for use in the present disclosure is a pharmaceutical composition for intravenous administration. Typically, pharmaceutical compositions for intravenous administration are solutions in sterile isotonic aqueous buffer. Generally, the ingredients are supplied either separately or mixed together in unit dosage form, for example, as a dry lyophilized powder or water free concentrate in a hermetically sealed container such as an ampule or sachette indicating the quantity of active ingredient. Where the pharmaceutical composition is to be administered by infusion, it can be dispensed with an infusion bottle containing sterile pharmaceutical grade water or saline.

Those skilled in the art are familiar with the principles and procedures discussed in widely known and available sources as Remington's Pharmaceutical Science (17th Ed., Mack Publishing Co., Easton, Pa., 1985), Goodman and Gilman's The Pharmaceutical Basis of Therapeutics (8th Ed., Pergamon Press, Elmsford, N.Y., 1990) and "Tratado de Farmacia Galenica", C. Fauli and Trillo, Luzan 5, S.A. de Ediciones, 1993 and in Remington<'>s Pharmaceutical Sciences (A.R. Gennaro, Ed.), 20th edition, Williams & Wilkins PA, USA (2000).

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered as early as possible after the onset of ischemia at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered as early as possible after the onset of ischemia at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered as early as possible after the onset of ischemia at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 15 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 20 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 30 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 45 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 15 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 20 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 30 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 45 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 15 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 20 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 30 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 45 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 15 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 20 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 30 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 45 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 15 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 20 minutes prior to reperfusion.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 30 minutes prior to reperfusion.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 15 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 15 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 15 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 20 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 20 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 20 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 30 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 30 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 30 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 45 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 45 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 45 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 15 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 15 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 15 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 20 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 20 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 20 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 30 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 30 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 30 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 45 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 45 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 45 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 15 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 15 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 15 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 20 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 20 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 20 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 30 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 30 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 30 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 45 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 45 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 45 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 15 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 15 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 15 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 20 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 20 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 20 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 30 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 30 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 30 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 45 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 45 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 45 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 15 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 15 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 15 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 20 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 20 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 20 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 30 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 30 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 30 minutes prior to reperfusion at a dosage ranging from 0.3 to 0.6 mg/kg body weight.

In some embodiments, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 45 minutes prior to reperfusion at a dosage ranging from 0.1 to 1 mg/kg body weight. In a preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 45 minutes prior to reperfusion at a dosage ranging from 0.2 to 0.8 mg/kg body weight. In a more preferred embodiment, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 45 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 15 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 15 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 15 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 20 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 20 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 20 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 30 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 30 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 30 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 45 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 45 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 60 minutes after the onset of ischemia and at least 45 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 15 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 15 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 15 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 20 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 20 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 20 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 30 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 30 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 30 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 45 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 45 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 45 minutes after the onset of ischemia and at least 45 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 15 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 15 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 15 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 20 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 20 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 20 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 30 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 30 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 30 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 45 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 45 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 30 minutes after the onset of ischemia and at least 45 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 15 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 15 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 15 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 20 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 20 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 20 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 30 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 30 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 30 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 45 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 45 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 20 minutes after the onset of ischemia and at least 45 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 15 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 15 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 15 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 20 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 20 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 20 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 30 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 30 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 30 minutes prior to reperfusion at a dosage ranging from 0.3 to 0.6 mg/kg body weight.

In some embodiments, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 45 minutes prior to reperfusion at a dosage ranging from 0.1 to 1 mg/kg body weight. In a preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 45 minutes prior to reperfusion at a dosage ranging from 0.2 to 0.8 mg/kg body weight. In a more preferred embodiment, ischemia-related damage is the damage arising after the onset of ischemia, but prior to reperfusion, the compound for use according to the present disclosure is atorvastatin (formula I) or the β-hydroxy acid form of simvastatin (formula II) and is to be administered by intravenous administration after the onset of ischemia, but prior to reperfusion, wherein the compound is to be administered not more than 15 minutes after the onset of ischemia and at least 45 minutes prior to reperfusion at a dosage ranging from 0.3 to 0.6 mg/kg body weight.

The compound for use according to the present disclosure may be administered alone or in combination with other drugs provided that at least one of the drugs is a compound for use according to the present disclosure.

"Infarct" relates to a localized area of ischemic cell death produced by hypoxia following occlusion of the arterial supply or venous drainage of a tissue or organ. More particularly, a myocardial infarction (MI), commonly known as heart attack is related to an event by which blood stops flowing properly to part of the heart, and the heart muscle is injured due to not receiving enough oxygen and nutrient supply. Usually an infarction is the result of blockage or occlusion of one of the coronary arteries due to the rupture or erosion of an atherosclerotic plaque and the superimposition of a thrombus. Important risk factors that may contribute to the presentation of myocardial infarction include a previous cardiovascular event, high total cholesterol levels, high blood levels of LDL cholesterol and triglycerides, low levels of HDL cholesterol, old age, tobacco smoking, diabetes, high blood pressure, lack of physical activity, obesity, chronic kidney disease, excessive alcohol consumption, and the use of cocaine and amphetamines. Methods to determine whether a subject has suffered from infarction are known in the art and include, without limitation, tracing electrical signals in the heart by an electrocardiogram (ECG), and testing a blood sample for substances associated with heart muscle damage, including creatine kinase (CK-MB) and troponin. ECG testing is used to differentiate between two types of myocardial infarctions based on the shape of the tracing. An ST section of the tracing higher than the baseline is called an ST elevation MI (STEMI) which usually requires more aggressive treatment. Methods to determine infarct size are known by the skilled person and include measurement of serum markers including creatine kinase (CK)-MB levels in a serum sample (Grande P et al. 1982 Circulation 65: 756-764), tissue staining with triphenyl tetrazolium chloride (Fishbein MC et al. 1981 Am Heart J 101(5): 593-600), technetium (Tc)-99m sestamibi single-photon emission computed tomography (SPECT) myocardial perfusion imaging, and magnetic resonance imaging (MRI) (Gibbons RJ et al. 2004 J Amer Coll Cardiol 44(8): 1533-1542). Those biomarkers of cardiac damage rise after few hours, whereas markers such as IMA, CFABP and myoglobin are known to increase early after ischemia.

"Inflammation", or "inflammatory response", relates to a set of changes occurring in a tissue that undergoes inflammation. In particular, inflammation relates to the biological response to harmful stimuli, including pathogens, damaged or lysed cells or irritants. Methods to determine inflammation are known in the art and include, without limitation, measure of erythrocyte sedimentation rate (ESR), wherein a higher ESR is indicative of inflammation, measure of C-reactive protein (CRP), wherein a higher level of CRP is indicative of inflammation, and leukocyte count (increased in inflammation).

Local inflammation of an ischemic tissue, e.g. ischemic myocardium, can be determined by molecular analysis (gene and protein expression) of inflammatory markers related to both innate and acquired immunity. Particularly, it is of interest to measure chemokines and cytokines involved in leukocyte activation and recruitment to the damaged area.

Systemic inflammation due to ischemia may affect all organs. Blood analysis of inflammatory markers (cytokines and chemokines) by the use of ELISA kits may provide a rapid analysis of the degree/level of systemic inflammation.

"Oxidative damage" relates to the biomolecular damage that can be caused by direct attack of reactive oxygen species during oxygen restoration. Oxidative damage may involve lipid peroxidation, oxidative DNA damage and oxidative damage to proteins. Methods to determine lipid peroxidation include, without limitation, MDA (malondialdehyde)-TBA (thiobarbituric acid) determination by HPLC, and quantification of isoprostanes (which are specific end products of the peroxidation of polyunsaturated fatty acids) by mass spectrometry. Methods to determine DNA oxidative damage include, without limitation, measure of 8-hydroxy-2'-deoxyguanosine (8-OH-dG). Methods to determine oxidative damage to proteins include, without limitation, quantification of individual aminoacid oxidation products including kynurenines (from tryptophan), bityrosine (which appears to be metabolically stable and can be detected in urine), valine and leucine hydroxides, L-dihydroxyphenylalanine (L-DOPA), ortho-tyrosine, 2-oxo-histidine, glutamate semialdehyde and adipic semialdehyde, as well as the carbonyl assay (involving measurement of protein carbonyl groups). The mitochondrial membrane potential (Δψιη) relates to the membrane potential in the form of proton gradient across the mitochondrial inner membrane. Methods for evaluation of mitochondrial membrane potential damage are known by the skilled person and include the use of fluorescent probes for monitoring membrane potential including the JC1 dye (Cell Technology) and the measure of overall fluorescence at excitation and emission wavelengths allowing the quantification of green (485 nm and 535 nm) and red fluorescence (550 nm and 600 nm). Prolonged ischemia of any tissue or organ is known to induce mitochondrial membrane potential damage.

"Apoptosis" is related to a regulated network of biochemical events which lead to a selective form of cell suicide, and is characterized by readily observable morphological and biochemical phenomena, such as the fragmentation of the deoxyribonucleic acid (DNA), condensation of the chromatin, which may or may not be associated with endonuclease activity, chromosome migration, margination in cell nuclei, the formation of apoptotic bodies, mitochondrial swelling, widening of the mitochondrial cristae, opening of the mitochondrial permeability transition pores and/or dissipation of the mitochondrial proton gradient. Methods to determine cell apoptosis are known by the skilled person and include, without limitation, assays that measure DNA fragmentation (including staining of chromosomal DNA after cell permeabilization), assays that measure the activation of caspases such as caspase 3 (including protease activity assays), assays that measure caspase cleavage products (including detection of PARP and cytokeratin 18 degradation), assays that examine chromatin chromatography (including chromosomal DNA staining), assays that measure DNA strand breaks (nicks) and DNA fragmentation (staggered DNA ends) (including active labeling of cell nick translation or ISNT and active labeling of cells by end labeling or TUNEL), assays that detect phosphatidylserine on the surface of apoptotic cells (including detection of translocated membrane component), assays that measure plasma membrane damage/leakage (including trypan blue exclusion assay and propidium iodide exclusion assay). Exemplary assays include analysis of scatter's parameters of apoptotic cells by flow cytometry, analysis of DNA content by flow cytometry (including DNA staining in a fluorochrome solution such as propidium iodide), fluorochrome labelling of DNA strand breaks by terminal deoxynucleotidyl transferase or TdT-assay, analysis of annexin-V binding by flow cytometry and TUNEL assay.

"Scar" relates to any mark left on a tissue following the healing of a wound or damage. Particularly, this term related to the marks left in ischemic tissue. In the context of the present disclosure, scar formation is derived from ischemia-related damage

According to the present disclosure, the ischemia causing the ischemia-related damage to be prevented/reduced according to the present disclosure occurs in an organ or tissue selected from the group consisting of brain, heart, kidneys, liver, large intestine, lungs, pancreas, small intestine, stomach, muscles, bladder, spleen, ovaries and testes. In a particular embodiment, the organ/tissue is selected from the group comprising heart, brain, kidney, intestine, pancreas, liver, lung, and skeletal muscle. In a more particular embodiment, the organ is heart.

The ischemia-related damage to be prevented/reduced occurs typically in the tissue where the ischemia with or without reperfusion occurs. In addition ischemia damage may lead to further ischemia-related damage in other tissues, as implied by the systemic inflammatory response triggered by the ischemia.

According to the present disclosure, the ischemia-related damage to be prevented/reduced according to the present disclosure occurs in an organ or tissue selected from the group consisting of brain, heart, kidneys, liver, large intestine, lungs, pancreas, small intestine, stomach, muscles, bladder, spleen, ovaries, testes and blood cells. In a particular embodiment, the organ/tissue is selected from the group comprising heart, brain, kidney, intestine, pancreas, liver, lung, skeletal muscle and blood cells. In a more particular embodiment, the ischemia damage to be prevented and/or reduced according to the present disclosure occurs in the heart.

In some embodiments, the ischemia-related damage to be prevented/reduced according to the present disclosure occurs in the heart and in blood cells (e.g. by systemic inflammatory effects). In some embodiments, the ischemia-related damage to be prevented/reduced according to the present disclosure occurs in the myocardium and in PBMCs (peripheral blood mononuclear cells).

As the skilled person in the art acknowledges, there are different causes that may cause ischemia and hence, ischemia-related damage. In some embodiments, ischemic damage is due to atherosclerosis, thrombosis, thromboembolism, lipid-embolism, bleeding, stent, surgery, angioplasty, bypass surgery, organ transplantation, total ischemia, vascular compression by a tumor, myocardial infarction, vasoconstriction, microvascular dysfunction or combinations thereof. In some embodiments, ischemia-related damage is produced during the reperfusion of the affected vessel.

"Atherosclerosis" relates to any stiffening of arteries secondary to atheroma or accumulation in the intima of the arterial wall of lipids, inflammatory cells (mostly macrophage/foam cells) and cell debris that are loaded with lipids, and vascular smooth muscle cells that have migrated from the media. The artery wall thickens as a result of the accumulation of calcium and fatty materials such as cholesterol and triglyceride. The elasticity of the artery walls is reduced and it may protrude towards the vascular lumen reducing blood flow. It also may become vulnerable to rupture and trigger thrombus formation (ischemic event).

"Thrombosis" relates to the formation of a blood thrombus inside a blood vessel because of the activation of the coagulation cascade and platelet activation/aggregation. The thrombus consequently obstructs the flow of blood through the circulatory system. "Thromboembolism" relates to the formation in a blood vessel of a clot (thrombus) that breaks loose and is carried by the blood stream to plug another vessel. The clot may plug a vessel in the lungs (pulmonary embolism), brain (stroke), gastrointestinal tract, kidneys, or leg.

"Lipid-embolism" or "fat embolism" refers to the often asymptomatic presence of fat globules in the lung parenchyma and peripheral circulation after long bone or other major trauma.

"Bleeding" relates to the process of losing blood or having haemostatic defects, especially surgically. In particular, internal bleeding occurs when there is damage to an artery or vein allowing blood to escape the circulatory system and collect inside the body. The internal bleeding may occur within tissues, organs, or in cavities of the body.

"Stent" relates to a dispositive such as a tube inserted into a natural passage/conduit in the body to prevent or counteract a disease-induced, localized flow constriction.

By "surgery" or "surgical treatment" is meant any therapeutic procedure that involves methodical action of the hand or of the hand with an instrument, on the body of a human or other mammal, to produce a curative or remedial.

"Angioplasty" relates to a technique of mechanically widening narrowed or obstructed arteries, the latter typically being a result of atherosclerosis. An empty and collapsed balloon on a guide wire, known as a balloon catheter, is passed into the narrowed locations and then inflated to a fixed size using water pressures some 75 to 500 times normal blood pressure (6 to 20 atmospheres). The balloon forces expansion of the inner white blood cell/clot plaque deposits and the surrounding muscular wall, opening up the blood vessel for improved flow, and the balloon is then deflated and withdrawn. A stent may or may not be inserted at the time of ballooning to ensure the vessel remains open.

"Bypass surgery" relates to a class of surgeries involving re-routing a tubular body part and includes cardiopulmonary bypass, partial ileal bypass surgery, ileojunal bypass, gastric bypass and vascular bypass such as coronary artery bypass surgery. Cardiopulmonary bypass (CBP) temporarily takes over the function of the heart and lungs during surgery, maintaining the circulation of blood and the oxygen content of the body. Partial ileal bypass surgery is a surgical procedure which involves shortening the ileum to shorten the total small intestinal length. The ileojejunal bypass is a surgery designed as a remedy for morbid obesity. A vascular bypass is a surgical procedure performed for inadequate or loss of blood flow to a region of the body. In particular, coronary artery bypass surgery, also known as coronary artery bypass graft (CABG) surgery, is a surgical procedure performed to relieve angina and reduce the risk of death from coronary artery disease.

By "transplantation" is meant a surgical procedure by which a cell, tissue or organ is transferred from a donor subject to a recipient subject or from one part of the body to another in the same subject. The "donor subject" is the subject who gives blood, cells, tissues, or an organ for another subject by blood transfusion or an organ transplant. The donor subject is a human or another mammal. The "recipient subject" is the subject who receives blood, cells, tissues, or an organ from another subject by blood transfusion or an organ transplant. The recipient subject is a human or another mammal. Transplanted tissues comprise, but are not limited to, bone tissue, tendons, corneal tissue, heart valves, veins and bone marrow. Transplanted organs comprise, but are not limited to, heart, lung, liver, kidney, pancreas and intestine. The particular surgical procedure of transplantation wherein the donor subject and the recipient subject are genetically non-identical members of the same species is known as allotransplantation. Thus, the term allotransplant (also known as allograft, allogeneic transplant or homograft) is related to the transplantation of cells, tissues or organs sourced from a genetically non-identical member of the same species as the recipient. The term "allotransplantable" refers to organs or tissues that are relatively often or routinely transplanted. Examples of allotransplantable organs include heart, lung, liver, pancreas, kidney and intestine. The particular surgical procedure of transplantation wherein the donor subject and the recipient subject are members of different species is known as xenotransplantation. Thus, the term xenotransplant (also known as xenograft, xenogeneic transplant or heterograft) is related to the transplantation of cells, tissues or organs sourced from a donor to a recipient, wherein donor and recipient are members of different species.

"Total ischemia" relates to ischemia in which arterial and/or venous blood supply is completely occluded (no flow). In a more particular embodiment, the ischemia-related damage is due to coronary obstruction (myocardial infarction). In another embodiment, the ischemia-related damage is due to reperfusion in relation to the ischemic damage.

"Myocardial infarction" (MI) involves an ischemic necrosis of part of the myocardium due to the obstruction of one or several coronary arteries or their branches. Myocardial infarction is characterized by the loss/death of functional cardiomyocytes, the myocardial tissue being irreversibly damaged. The myocardium, or heart muscle, suffers an infarction when advanced coronary disease exists, in a particular case this occurs when an atheromatous plaque located inside a coronary artery ulcerates or ruptures, triggering thrombus formation and an acute obstruction of that vessel, resulting in ischemia.

The subject according to the present disclosure is any subject for whom ischemia-related damage to an organ or tissue is to be prevented and/or reduced. In a particular embodiment, the subject is a mammal, including humans, domestic animals, farm animals, and zoo, sports, or pet animals such as dogs, cats, guinea pigs, rabbits, rats, mice, horses, cattle, cows, and so on. In a more preferred embodiment of the present disclosure, the subject is a human being.

In a particular embodiment, the subject suffers from dyslipidimia. In the most preferred embodiment of the invention (as regards the patient group), the subject suffers from hypercholesterolemia. Hypercholesterolemia relates to a pathological condition characterized by the presence of high blood cholesterol levels, particularly total cholesterol and LDL levels. As used herein, the term total cholesterol refers to the sum of the sub-fractions of LDL (low density lipoprotein), HDL (high density lipoprotein) and VLDL (very low density lipoprotein) cholesterol. The person skilled in the art knows the reference values of the blood total cholesterol and LDL levels, which will vary depending on different circumstances such as age, gender, etc., and will know how to determine in each case if a subject suffers hypercholesterolemia.

According to the present disclosure, a subject is considered as suffering from dyslipidimia when the subject has an abnormal amount of lipids (e.g., triglycerides, cholesterol and/or fat phospholipids) in the blood. In developed countries, most dyslipidemias are hyperlipidemias; that is, an elevation of lipids in the blood. Hyperlipidemias are divided into primary and secondary subtypes. Primary hyperlipidemia is usually due to genetic causes (such as a mutation in a receptor protein), while secondary hyperlipidemia arises due to other underlying causes such as diabetes, conditions involving the intersection of genetics and lifestyle issues.

According to the present disclosure, a subject is considered as suffering from hypercholesterolemia if total cholesterol levels are found above 200mg/dL and/or low-density lipoprotein cholesterol is found above 100mg/dL.

### 3. Use for the manufacture of a medicament

In a further aspect, the present disclosure relates to the use of a compound for the manufacture of a medicament for the prevention/reduction of ischemia-related damage in a subject, wherein said compound is a statin, wherein said compound is to be administered to said subject by intravenous administration after the onset of ischemia, but prior to reperfusion, and wherein said compound is to be administered as early as possible after the onset of ischemia.

In some embodiments, said compound, said prevention/reduction, said ischemia, said ischemia damage, said reperfusion damage, said ischemia-related damage, said subject, said statin and/or said administration are as defined above.

### 4. Methods of treatment

In a further aspect, the present disclosure relates to a method of treatment comprising the step of administering to a subject suffering from ischemia a pharmaceutically effective amount of a compound, wherein said compound is a statin, wherein said compound is administered to said subject by intravenous administration after the onset of ischemia, but prior to reperfusion, and wherein said compound is administered as early as possible after the onset of ischemia.

In some embodiments, said compound, said ischemia, said ischemia damage, said reperfusion damage, said ischemia-related damage, said pharmaceutically effective amount, said subject, said statin and/or said administration are as defined above.

The invention is detailed below by means of the following examples which are merely illustrative and by no means limiting the scope of the present disclosure.

### EXAMPLES

All the study protocols have been approved by the institutional ethics committee (CSIC-ICCC) and all animal procedures were carried out in strict accordance to the guidelines from Directive 2010/63/EU of the European Parliament on the protection of animals used for scientific purposes or the National Institute of Health (NIH) guidelines (NIH Publication No. 85-23, revised 1996). In addition, we have followed the ARRIVE guidelines and committed to the 3Rs of laboratory animal research.

### Example 1

### Swine model for ischemia

Experiments with a swine model of myocardial ischemia mimicking myocardial infarction were carried out as shown in **Figure 1****.**

Ischemia was experimentally induced in regular-chow fed pigs (≈ 40 kg) by closed-chest myocardial infarction induction. to that end, animals were sedated and anesthesia was maintained with isofluorane during the whole experimental procedure. Cardiac rhythm, arterial oxymetry, and arterial pressure were continuously monitored during the procedure. Just before starting the procedure a perfusion of amiodarone and lidocaine was initiated as prophylaxis for malignant left ventricular arrhythmias. All animals of the study were subjected to the same antiarrhythmic pharmacological approach allowing a direct comparison between the groups. By percutaneous approach and fluoroscopy guidance ischemia was induced by complete coronary balloon occlusion of the mid-left anterior descending coronary artery. Contrast angiographies were performed to ensure total coronary occlusion.

After 15 min of ischemia, animals either received an intravenous infusion of atorvastatin (0.3 mg/kg) or vehicle. Drug dosages had been determined on the basis of the loading dose (80 mg) in the setting of acute coronary syndrome and/or previous PCI (percutaneous coronary intervention) and have been converted to the pig dose according to body surface area. Control animals were given intravenous placebo (saline) at 15 min of ischemia.
Blood samples (serum) for further assessment of biomarkers of cardiac damage were taken at baseline (i.e. before MI induction or at time = 0 min), and at 30 min, 60 min and 90 min after MI induction (ischemia).
Blood samples were also collected in EDTA at baseline (prior to ischemia) and after 90 min of ischemia from the femoral artery for PBMC isolation by the Ficoll method.

Upon taking the 90 min blood sample, the pigs were sacrificed without reperfusion to obtain myocardial tissue for molecular studies and histology.

### Example 2

In this example, it was tested whether intravenous administration of a single dose of atorvastatin after the onset of coronary occlusion protects against ischemia-related damage (design in **Example 1**). The experiment was carried out in 7 animals per group (atorvastatin-treated vs. vehicle-control).

The concentration of IMA (Ischemic Modified Albumin), CFABP (Cardiac Fatty Acid Binding Protein) and Myoglobin was determined by the respective pig ELISA kits in the blood samples taken 30 min, 60 min and 90 min after MI induction and compared to their baseline values (prior to ischemia induction). These proteins were chosen based on the rationale that they are early markers for cardiac damage assessment due to ischemia.

As shown in the example, at all three time points examined, the intravenous administration of atorvastatin significantly reduced IMA **(****Figure 2A****),** CFABP **(****Figure 2B****)** and Myoglobin **(****Figure 2C****)** blood levels as compared to controls. The left panels clearly reflect the overall reduction in the three cardiac damage markers in atorvastatin-treated animals vs controls when combining the three-time points. Finally, as seen in **Figure 2D****,** there was an overall reduction of more than 50% for IMA levels and more than 75% for CFABP and myoglobin levels in the atorvastatin-treated animals as compared to the absence of treatment.

Thus, this example demonstrates that intravenous administration of the statin atorvastatin early after induction of ischemia significantly lowers the levels of ischemic damage markers as compared to individuals not treated with the statin.

### Example 3

In this example, it was tested whether intravenous administration of statin atorvastatin has an effect on RhoA activation in the ischemic myocardium of the pigs described in **Example 1** and **Example 2.**

After the ischemic period, the animals' hearts were arrested with potassium chloride and rapidly excised. Tissue samples obtained from the ischemic myocardium of all animals were pulverized and homogenized in lysis buffer for protein isolation and total RhoA assessment. In addition, the separation of the membrane and cytosolic fractions was performed by a modification of the lysis buffer protocol. Briefly, tissue powder was homogenized (1 mol/L NaCl, 20 mmol/L Tris pH 7.4, 1 mmol/L DTT and protease inhibitors) and centrifuged. The supernatant was collected (referred to as the cytosolic fraction) and pellets were resuspended, and the membrane proteins were extracted by incubation in 500 mmol/L Tris-HCI pH 7.4, 20% sodium dodecyl sulfate, 100 mmol/L sodium orthovanadate, and protease inhibitors. The extract was centrifuged and the supernatant was collected as the membrane fraction.

Protein concentration (in total myocardial tissue, in the cytoplasm fraction and in the membrane fraction) was quantified by the Pierce method. RhoA (Santa Cruz) was determined in total and cytosolic and membrane fractions of the ischemic cardiac tissue of all animals by Western Blot analysis. The intensity of the Western blot bands was detected with Chemi-Doc (Bio-Rad) and they were quantified with the Quantity one Software (Bio-Rad). Intensities are expressed in arbitrary units (AU).

As can be seen from **Figure 3****,** the total amount of RhoA protein in the ischemic myocardium was not affected by statin treatment after the onset of ischemia **(****Figure 3A****).** However, statin treatment significantly affected RhoA activation. As such, non-treated control animals showed a lower RhoAcyt/mb ratio indicative of higher RhoA bound to the membrane (active form) **(****Figure 3B****).** In contrast, in statin-treated animals RhoA was mainly localized in the cytoplasm (inactive).

Thus, this example indicates that a single dose of statin (e.g., atorvastatin) after the onset of ischemia inhibits RhoA activation in the ischemic myocardium of infarcted pigs.

### Example 4

In this example, it was tested whether whether the cardioprotective effects associated with statin treatment involve RhoA inhibition.

The experiment was carried out with a mouse model for myocardial ischemia, wherein the ischemia was caused by coronary ligation **(****Figure 4A****).**

Complete LAD coronary artery ligation was performed as follows. Male CH3 mice (8-10 weeks old, weighing 25-30 g) were intubated and anesthesized with a mixture of O_{2/}isoflurane and mechanically ventilated (rate 90 breaths/min, tidal volume 0.1 mL). Rectal temperature was continuously monitored throughout the surgery and maintained within 37-38 °C using a heat pad and heat lamp. An anterior thoracotomy was performed; the heart was exposed and the LAD coronary artery was occluded with an intramural stitch (7-0 silk suture; consistent ligation of the left coronary artery as it emerges from under the left atrium) for 45 minutes. Success of complete coronary ligation was verified by electrocardiographic visualization of the ST-elevation-MI pattern that was monitored by high frequency ultrasounds with the Vevo2100 from Visualsonics and by the visualization of a pale, hypokinetic ventricle distal to the site of occlusion.

15 minutes after coronary ligation (i.e. 15 min after the onset of ischemia), a single dose of atorvastatin (0.3 mg/kg; n=6), β-OH-simvastatin (0.3 mg/kg; n=6), the specific RhoA inhibitor CCG1423 (Sigma; 0.15 mg/kg; n=6) or PBS volume for vehicle/controls (n=6) were administered intraperitoneally. Ischemia was continued for another 30 min after administration of the single dose (i.e. altogether 45 min of ischemia), then mice were sacrificed and the heart was carefully excised for infarct size analysis.

Infarct size was determined by morphometric analysis. For this purpose, all mouse hearts were immersed in fixative solution (4% paraformaldehyde), embedded in OCT compound and cross-sectioned from apex to base (10 µm thick sections 200 µm distanced). Sections were stained with haematoxylin and eosin and morphometric infarct size analysis was determined using image analysis software (ImageJ, NIH). Infarct size was calculated by the sum of myocardial infarct areas between sections and expressed as a percentage of total LV wall. Three measurements per section were determined.

In addition, myocardial oxidative damage was also assessed. For this purpose, ischemic myocardial tissue was cut into 5-µm-thick slices for 8-OH-dG (Abcam ab48508) staining, a measurement indicative of oxidative stress-induced DNA-damage. Staining was calculated by a single blinded observer from an average of 5-fields/animal as % of stained area. Images were captured by Nikon Eclipse 80i microscope and digitized by Retiga 1300i Fast camera.

As can be seen from the histological pictures in **Figure 4B****,** the size of infarction was clearly reduced by around 50% by statins (atorvastatin and β-OH-simvastatin) as well as for the RhoA inhibitor CCG1423 as compared to vehicle-control.

Statin and CCG1423 administration was also associated with lower oxidative damage (70% reduction) within the ischemic myocardium **(****Figure 4C****).**

Thus, this example shows that the cardioprotective effects of statins are mediated, in part, through RhoA inhibition.

### Example 5

In this example, the effect of intravenous administration of a statin during ischemia on mRNA levels of cardioprotective cellular proteins [AMPK (AMP-activated protein kinase) and eNOS (endothelial nitric oxide synthase)] and apoptosis-related markers p53 and caspase-3 in both the ischemic and non-ischemic myocardium was determined. The study was carried out in the myocardium samples obtained from pigs as described in **Example 1** and **2.**

To this end, tissue samples obtained from the ischemic myocardium and non-ischemic myocardium of all animals were pulverized and homogenized in Tripure (Roche) for RNA isolation. We performed transcriptomic analysis to assess by real-time PCR the gene expression of AMPK, eNOS, P53 and caspase-3. The threshold cycle values were determined and normalized to the housekeeping gene 18SrRNA.

As shown in **Figure 5****,** atorvastatin did not exert any changes in the mRNA levels of AMPK, eNOS, p53 and caspase-3 in both ischemic and non-ischemic myocardium as compared to controls.

Thus, atorvastatin treatment early after the ischemic insult does not modify mRNA levels of cardioprotective- and apoptotic- related markers assessed at 90 min post-ischemia.

### Example 6

In this example, the effect of intravenous administration of a statin early after the onset of ischemia on AMPK and eNOS activation (proteins known to exert cardioprotective effects) was examined.

The study was carried out in the myocardial samples from pigs as described in **Example 1** and **2.**

To this end, tissue samples obtained from the ischemic myocardium and non-ischemic myocardium of all animals were pulverized and homogenized in lysis buffer. We assessed AMPK, AMPK phosphorylated at Thr172 (P-AMPK) or activated AMPK, eNOS, and eNOS phosphorylated at Ser1177 (P-eNOS) or activated eNOS. The degree of AMPK and eNOS activation was evaluated by assessing P-AMPK to total AMPK ratio and P-eNOS to total eNOS ratio, respectively. The intensity of the bands was calculated by densitometry and expressed as arbitrary units (AU).

As can be seen from **Figure 6A****,** levels of P-AMPK were significantly increased in the ischemic myocardium of atorvastatin-treated animals as compared to controls. No changes were observed in P-AMPK in the non-ischemic cardiac tissue between atorvastatin-treated animals and controls showing protein levels comparable to that found in the ischemic myocardium of atorvastatin-treated animals. No changes were detected in the total protein content of AMPK in the entire heart in both animal groups. Accordingly, the ratio P-AMPK/AMPK was significantly increased in atorvastatin-treated animals. These observations are shown in the representative western blot bands.

As can be seen from **Figure 6B****,** atorvastatin treatment did not further enhance (but also did not diminish) ischemia-induced eNOS activation. Again, a representative image of the western blot bands is provided.

Thus, already a single dose of the statin atorvastatin administered early after the onset of ischemia preserves the activation of the cardioprotective protein AMPK, while it does not affect the activation status of eNOS.

### Example 7

In order to determine whether atorvastatin-related activation of AMPK was involved in the cardioprotective effects afforded by the statin we carried out an experiment in a mouse model of myocardial ischemia. The animal model of coronary ligation is detailed in **Example 4** and **Figure 4****.**

In this case an AMPK inhibitor (compound C; 30 mg/kg; dissolved in DMSO) was given intraperitoneally prior to atorvastatin administration (0.3 mg/kg) in order to block any potential effect of the statin on AMPK activation. Atorvastatin was administered at 15 min of ischemia and after 30min (total ischemic period 45 min) mice were sacrificed and hearts were processed for myocardial infarct size and oxidative assessment as detailed in

### Example 4.

Histological analysis of infarct size **(****Figure 7A****)** revealed that blockade of AMPK activation by Compound C abolished the infarct size reducing properties achieved by atorvastatin administration shortly after induction of ischemia. In addition **Figure 7B** indicates that AMPK activation also protected against oxidative damage [assessed by 8-hydroxyguanosine staining (8-OHdG)] since administration of Compound C abolished these protective effects.

Thus, the cardioprotective effects of atorvastatin against ischemic injury (including lower infarct size and oxidative damage) are partly mediated though AMPK activation.

### Example 8

This example was carried out to address the question whether atorvastatin administration during ischemia prevents apoptosis execution in the ischemic myocardium of pigs.

Myocardial tissue of all pigs included in **Example 1** and **Example 2** was obtained, pulverized and processed with lysis buffer for protein isolation as detailed in **Example 6.** Protein was quantified by the Pierce method and Western Blot analysis was performed to assess the levels of phosphorylated p53 (Ser15), total p53, cleaved caspase-3 and total caspase-3. The ratios phosphorylated p53/total p53 and cleaved Casp3/total caspase3 were calculated to ascertain how much of protein was activated.

From the data in **Figure 8A****,** it can be seen that atorvastatin administration early after ischemia reduced the activation of p53 within the ischemic myocardium whereas exerted no effects on p53 activation within the non-ischemic myocardium. No changes were observed in total p53 levels within the entire myocardium. Accordingly, the P-p53/p53 ratio was significantly reduced in the ischemic myocardium of atorvastatin-treated pigs.

Regarding the impact of atorvastatin treatment on caspase-3 activation, as observed in **Figure 8B****,** atorvastatin administration reduced the levels of cleaved (active) caspase 3 within the ischemic myocardium. Since no changes were reported for total caspase-3, atorvastatin consequently reduced the cleaved caspase-3/total caspase-3 ratio, as can be seen from the representative Western Blot bands of **Figure 8B****.**

Thus, atorvastatin administration early after ischemia reduces the levels of apoptosis execution within the ischemic myocardium assessed by decreased levels of both phosphorylated p53 and cleaved caspase 3.

### Example 9

This example investigated whether administration of atorvastatin early after ischemia induction exerts anti-inflammatory effects on the myocardium. To this end, the effect of atorvastatin on the expression of monocyte chemoattractant protein-1 (MCP-1/CCL2) was evaluated at an mRNA level and protein levels since MCP-1 is one of the key chemokines that regulates leukocyte (particularly monocytes) migration and infiltration. Migration of monocytes from the blood stream across the vascular endothelium occurs early after an ischemic insult.

The myocardium of pigs included in **Example 1** and **Example 2** was obtained, pulverized and processed with Tripure or lyses buffer for mRNA and protein isolation as detailed in **Example 5** and **Example 6,** respectively. Both the mRNA levels and protein expression of MCP-1 were assessed. The threshold cycle (Ct) values were determined and normalized to the housekeeping gene 18SrRNA in order to adjust for equal amounts of RNA whereas β-actin was used as protein loading control.

As can be seen from **Figure 9****,** atorvastatin treatment significantly reduced both MCP-1 mRNA and protein expression in the ischemic myocardium as compared to non-treated pigs.

No changes were detected on MCP-1 expression in the non-ischemic myocardial tissue.

Thus, intravenous administration of atorvastatin shortly after induction of ischemia has anti-inflammatory effects on ischemic cardiac tissue.

### Example 10

In this example, the question addressed was whether the myocardial anti-inflammatory effects of atorvastatin also extend systemically to circulating leukocytes, particulary peripheral blood mononuclear cells (PBMC).

To this end, 20 ml of blood were collected into EDTA tubes at three tested time-points (0, 30, and 90 min) in all animals. All blood samples were immediately subjected to Ficoll-paque Plus (Amersham Biosciences) density gradient centrifugation to isolate PBMCs. Samples were counted and then directly frozen in liquid nitrogen and stored at -80°C until mRNA and protein isolation as described in **Example 5** and **Example 6.** The mRNA expression level of the inflammatory markers related to innate immunity TLR4 (Toll-like receptor 4) and MCP-1, as well as TF (tissue factor) were determined by Real Time PCR analyses whereas protein expression of MCP-1 was also assessed. The threshold cycle (Ct) values were determined and normalized to the housekeeping gene 18SrRNA in order to adjust for equal amounts of RNA whereas Ponceau red was used for protein loading control.

As shown in **Figure 10A** atorvastatin treatment did not modify TLR4 or TF PBMC expression either prior- (time 0 min) or post- (time 90 min) ischemic induction. In contrast, atorvastatin markedly attenuated the ischemia-triggered increase in MCP-1 expression at both gene and protein levels.

Thus, this example shows that the anti-inflammatory effects of atorvastatin extend beyond the ischemic tissue and also extend to the systemic circulation by preventing ischemia-related MCP-1/CCL2 induction in circulating PBMCs.

### Example 11

In this example, the question addressed was whether a single intravenous dose of atorvastatin early after ischemia reduces myocardial scar formation assessed 30 days after ischemia induction. The experiment was carried out with a rat model for ischemia, wherein the myocardial ischemia was caused by persistent coronary artery ligation **(****Figure 11A****).**

The study was performed in male SD rats (8-10 weeks old, weighing 250-300 g; Jackson Laboratory). MI was induced by LAD coronary artery ligation for 45min. After 15 min of ischemia rats were randomly given an intraperitoneal injection of 0.3 mg/kg of atorvastatin or equal PBS volume for vehicle/controls (n=6). Thirty minutes after atorvastatin administration (45 min of ischemia) animals were allowed to recover and kept for the following 30 days and then sacrificed. Afterwards hearts were carefully excised for morphometric assessment of infarct size.

The results of this experiment are shown in **Figure 11B****.** As can be seen from the data the single dose of atorvastatin 15 min after the onset of ischemia resulted in a significant 60% reduction in scar size assessed 30 days post-ischemia induction as compared to vehicle-controls.

Thus, atorvastatin administration early after ischemia induction exerts protective effects that translate into a lower myocardial scarring 1 month after ischemia induction.

### Example 12

In this example, the question to be addressed was whether a single intravenous dose of atorvastatin early after induction of ischemia reduces myocardial damage in the presence of co-morbid conditions such as hypercholesterolemia, the most common cardiovascular risk factor found in patients suffering from myocardial infarction.

To this end, 14 pigs were fed for 10 days a Western-type hypercholesterolemic diet [20%-saturated fat (beef tallow), 2%-cholesterol, 1%-cholic acid]. This hyperlipidemic diet contains 24.82% proteins, 64.20%of carbohydrates, 10.98% of fats and 16,834.7 kJ. Intake of this fat-rich diet for 10 days raises cholesterol to levels comparable to that found on dyslipidemic humans and induces endothelial dysfunction.

After this 10 day diet period animals underwent a closed-chest 90 min mid-left anterior descending (LAD) coronary balloon occlusion and the same experimental design described in **Example** 1. Briefly, 15 min after ischemia induction, animals (n=7 animals/group) were intravenously administered atorvastatin (0.3 mg/kg) and then blood was withdrawn at 30, 60 and 90 min of ischemia in order to determine early markers of cardiac damage (IMA, MFABP, myoglobin).

As can be seen from the data, a single dose of atorvastatin 15 min after the onset of ischemia resulted in a significant reduction in all three myocardial damage-related markers. As such, atorvastatin intravenous administration resulted in a significant reduction of IMA **(****Figure 12A****),** CFABP **(****Figure 12B****)** and Myoglobin **(****Figure 12C****)** already observed at 30 min after ischemia induction (15 min after atorvastatin administration). Percentage of reduction was around 60-75% for all three markers **(****Figure 12D****).**

Thus, this experiment provides evidence that intravenous administration of atorvastatin early after ischemia induction protects the myocardium against ischemic damage even in the presence of high cholesterol levels.

### Example 13

In this example, the question to be addressed was whether a single intravenous dose of atorvastatin early after ischemia reduces neutrophil recruitment to the ischemic damaged area in hypercholesterolemic pigs.

To this end, ischemic myocardial tissue of all hypercholesterolemic pigs included in **Example 12** was obtained, embedded in OCT, and stained for neutrophils detection (anti-Neutrophil Elastase; Abcam). Staining was calculated by a single blinded observer from an average of 5-fields/animal as % of stained area. Images were captured by Nikon Eclipse 80i microscope and digitized by Retiga 1300i Fast camera.

As can be seen from the data (Figure 13), a single intravenous dose of atorvastatin 15 min after the onset of ischemia resulted in a significant reduction in neutrophil myocardial infiltration (57% reduction) as compared to controls.

Thus, intravenous administration of atorvastatin in pigs early after ischemia induction limits neutrophil recruitment to the ischemic damaged myocardium in the presence of dyslipidemia.

### Example 14

In this example, the question to be addressed was whether administration of statin during the ischemic insult limits reperfusion damage associated to ischemia accordingly reducing the overall cardiac damage.

Pigs were fed a hypercholesterolemic diet as in **Example 11.** After this 10-day diet period animals underwent a closed-chest 90 min mid-left anterior descending (LAD) coronary balloon occlusion. After 75 min of ischemia animals were randomized to intravenous infusion (femoral vein) of an active β-hydroxy acid derivative of simvastatin (β-OH-S; 0.3 mg/kg; n=12) or vehicle (0.9% NaCl; n=12). At minute 90, the balloon was deflated to achieve coronary artery reperfusion. Animals were reperfused for 2.5 h. Infarct size was determined morphometrically by Evan's Blue staining. To that end, 2.5 h post-reperfusion Evan's Blue dye was injected in the anesthetized pigs through the left atrium to outline the area-at-risk (AAR) after which the animals' hearts were arrested with potassium chloride and rapidly excised. Hearts were sectioned into 6 transverse slices parallel to the atrioventricular ring. Consecutive slices were alternatively collected for infarct size analysis (triphenyl tetrazolium chloride) and molecular studies of the ischemic and non-ischemic myocardium. Tissue samples obtained from the ischemic and non-ischemic myocardium all animals were pulverized and homogenized in Tripure® or lysis buffer for RNA and protein isolation, respectively. Protein levels of apoptosis markers (truncated-caspase-3) was assessed. Paraffin-embedded ischemic myocardial tissue of all animals was cut into 5-µm-thick slices for 8-hydroxyguanosine staining (Abcam ab48508), a measurement indicative of oxidative stress-induced DNA-damage, and OCT-embedded ischemic myocardial tissue of all animals was stained for neutrophil detection (anti-Neutrophil Elastase; Abcam). Staining was calculated by a single blinded observer from an average of 5-fields/animal as % of stained area. Images were captured by Nikon Eclipse 80i microscope and digitized by Retiga 1300i Fast camera. In addition, mitochondrial membrane potential (ΔΨm) was measured in the ischemic myocardium of all animals by flow cytometry using the ratiometric dye 5,5',6,6'-tetrachloro-1,1',3,3'-tetraethylbenzimidazol carbocyanine iodide (JC-1; Molecular Probes). Values were expressed as red fluorescence activity. Isolated mitochondria treated with valinomycin (Sigma), which decreases ΔΨm, were used as control (data not shown).

As shown in **Figure 14****,** β-OH-S infusion during ischemia resulted in a decreased size of myocardial damage (ischemic damage+reperfusion damage) assessed 2.5 h after reperfusion **(****Figure 14A****).** In addition, β-OH-S infusion during ischemia significantly reduced the expression of cleaved caspase-3 **(****Figure 14B****),** limited the recruitment of neutrophils **(****Figure 14C****),** decreased oxidative stress **(****Figure 14D****)** and preserved mitochondrial potential **(****Figure 14E****)** in the ischemic myocardial tissue of all atorvastatin-treated animals.

Thus, infusion of β-OH-S during ischemia lessens reperfusion damage related to ischemia, overall limiting the myocardial damage.

## Claims

1. A compound for use in the prevention and/or reduction of ischemia-related damage in a subject, wherein said compound is a statin,wherein said compound is to be administered to said subject by intravenous administration after the onset of ischemia, but prior to reperfusion, wherein the compound is to be administered as early as possible after the onset of ischemia.

2. The compound for use according to claim 1, wherein ischemia-related damage is the damage caused by ischemia during the ischemic period after the onset of ischemia, but prior to reperfusion.

3. The compound for use according to any of claims 1 or 2, wherein the compound is to be administered in a single dose.

4. The compound for use according to any of claims 1 to 3, wherein the compound is to be administered not more than 60 minutes after the onset of ischemia, preferably not more than 45 minutes after the onset of ischemia, more preferably not more than 30 minutes after the onset of ischemia, more preferably not more than 20 minutes after the onset of ischemia, more preferably not more than 15 minutes after the onset of ischemia.

5. The compound for use according to any of claims 1 to 4, wherein the compound is to be administered at least 15 minutes prior to reperfusion, preferably at least 20 minutes prior to reperfusion, more preferably at least 30 minutes prior to reperfusion, more preferably at least 45 minutes prior to reperfusion.

6. The compound for use according to any of claims 1 to 5, wherein the compound is selected from the compounds listed in the following Table:
| **Statin and formula thereof** | **Further related statins** |
|---|---|
| 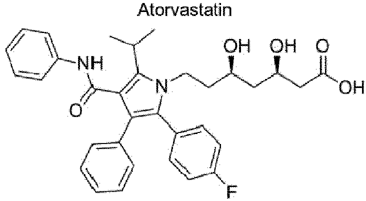 | Beta-oxidized atorvastatin, unsaturated beta-oxidized atorvastatin, beta-oxidized hydroxy atorvastatin, 4-hydroxy atorvastatin para-hydroxy atorvastatin acid, atorvastatin lactone, atorvastatin acyl glucuronide, 2-hydroxy atorvastatin ortho-hydroxy atorvastatin acid, 4-hydroxy atorvastatin acyl glucuronide, 4-hydroxy atorvastatin lactone and 2-hydroxy atorvastatin lactone. |
| 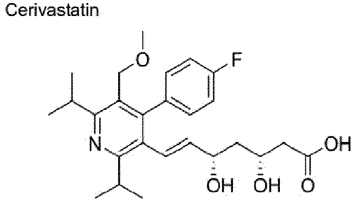 | Cerivastatin 6-O-glucuronide, cerivastatin 3-O-glucuronide, cerivastatin 4-O-glucuronide, cerivastatin lactone |
| 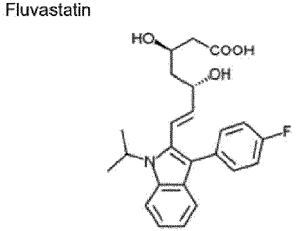 | 5-hydroxy fluvastatin, 6-hydroxy fluvastatin and N-deisopropyl fluvastatin. |
| 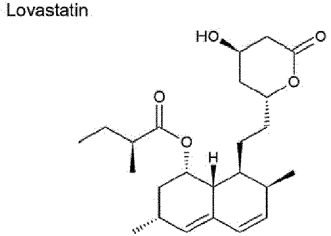 | Lovastatin acid, 3'-hydroxylovastatin, 6'-exomethylenelovastatin and 6'-beta-hydroxylovastatin and 3"β-hydroxylovastatin. |
| 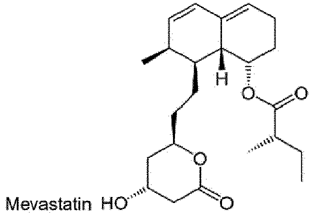 | |
| 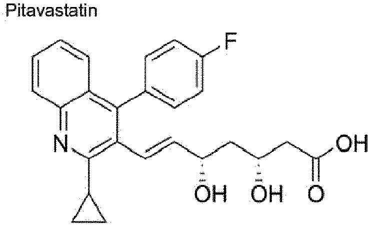 | Pitavastatin lactone, 3-keto pitavastatin, pitavastatin keto lactone, hydroxy pitavastatin lactone, 3-dehydroxy pitavastatin O-glucuronide, 5-keto pitavastatin acyl glucuronide, 5-keto pitavastatin O-glucuronide and 3-dehydroxy pitavastatin acyl glucuronide. |
| 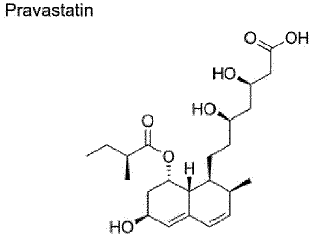 | 3"(S)-hydroxytetranorpravastatin, 3'-alpha-5',6'-epoxyisopravastatin, 3'-alpha-7-hydroxyisopravastatin, 3'-ketopravastatin-5,6-diol, 3"(S)-hydroxypravastatin, 3'-alpha-isopravastatin, 3'-alpha-5'-beta, 6'-beta-trihydroxypravastatin, 6'-epipravastatin, pravastatin glucuronide, desecylpravastatin, 4'-alpha-5-beta-epoxypravastatin, pravastatin 4a'alpha-glutathione conjugate, pravastatin lactone, pravastatin sulfate, |
| 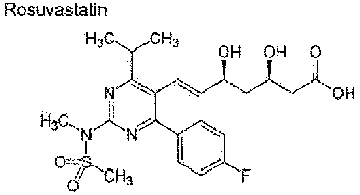 | Rosuvastatin lactone, N-demethylrosuvastatin and rosuvastatin acyl glucuronide. |
| 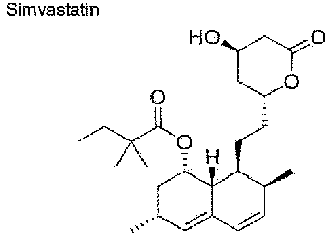 | 3',5'-dihydrodiolsimvastatin, 6'-beta-hydroxymethylsimvastatin, 6'beta-carboxysimvastatin, 6'-beta-hydroxysimvastatin, 6'-exomethylenesimvastatin, simvastatin acid terivastatin, simvastatin acid glucuronide and 3'-hydroxysimvastatin |
wherein, preferably, the compound is selected from the group consisting of atorvastatin, the β-hydroxy acid form of simvastatin, simvastatin, cerivastatin, fluvastatin, lovastatin, mevastatin, pitavastatin, pravastatin and rosuvastatin, wherein, more preferably, the compound is atorvastatin (formula I) or the β-hydroxy acid form of simvastatin of formula II, wherein, most preferably, the compound is atorvastatin (formula I) or the β-hydroxy acid form of simvastatin of formula III:

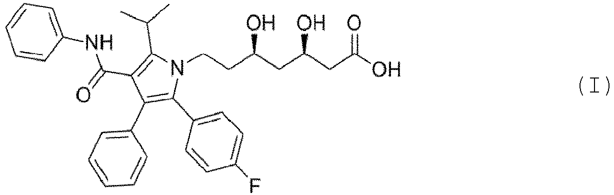

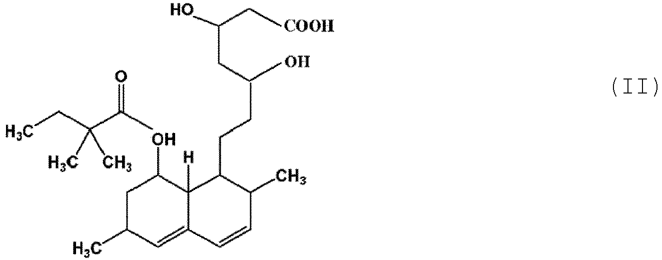

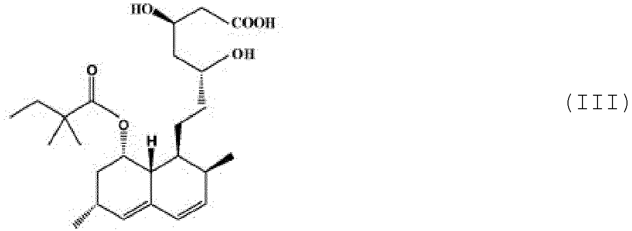

7. The compound for use according to any of claims 1 to 6, wherein the compound is to be administered at a dosage in the range of from 0.1 to 1 mg/kg body weight, preferably from 0.2 to 0.8 mg/kg body weight.

8. The compound for use according to any of claims 1 to 7, wherein the ischemia is produced in a tissue or organ selected from the group consisting of heart, brain, kidney, intestine, pancreas, liver, lung, skeletal muscle and combinations thereof, wherein, preferably, the ischemia is produced in the heart.

9. The compound for use according to any of claims 1 to 8, wherein ischemia-related damage is due to an ischemia caused by atherosclerosis, thrombosis, thrombo-embolism, lipid-embolism, bleeding, stent, surgery, angioplasty, bypass surgery, organ transplantation, stress-induced cardiomyopathy (Takotsubo syndrome), vasoconstriction, ST-elevated myocardial infarction, non-ST-elevated myocardial infarction, unstable angina, or a combination of two or more of these, wherein, preferably, the ischemia is related to ST-elevated myocardial infarction, non-ST-elevated myocardial infarction or unstable angina.

10. The compound for use according to any of claims 1 to 9, wherein the ischemia-related damage consists of at least one damage selected from the group consisting of myocyte cell death (preferably through necrosis and/or apoptosis, more preferably through necrosis), damage caused by intracellular pH acidification due to ischemia and/or damage caused by an inflammatory response due to ischemia that is triggered by ischemia and further amplified during reperfusion.

11. The compound for use according to any of claims 1 to 10, wherein the ischemia-related damage comprises or consists of damage to myofibrils caused by intracellular proteases which causes hypercontracture and/or contracture band necrosis.

12. The compound for use according to any of claims 1 to 11, wherein the prevention/reduction of ischemia-related damage comprises or consists of a reduction of the infarct size caused by ischemia, preferably a reduction of myocardial damage,
and/or wherein the prevention/reduction of ischemia-related damage leads to a reduction of tissue damage due to cell death triggered by ischemia/reperfusion, preferably due to cell death triggered by ischemia,
and/or wherein the prevention/reduction of ischemia-related damage comprises or consists of a prevention or reduction of the inflammatory response in the ischemic tissue/organ, and/or wherein the prevention/reduction of ischemia-related damage comprises or consists of the reduction of the inflammatory response in blood cells, preferably in leukocytes, triggered by ischemia and/or reperfusion, preferably triggered by ischemia.

13. The compound for use according to any of claims 1 to 12, wherein the prevention/reduction of ischemia-related damage comprises or consists of a reduction in scar size in the post-ischemic tissue, preferably in post-ischemic cardiac tissue.

14. The compound for use according to any of claims 1 to 13, wherein the subject is a human.

15. The compound for use according to any of claims 1 to 14, wherein the subject suffers from dyslipidemia, wherein, preferably, said dyslipidemia is hypercholesterolemia.

## Patentansprüche

1. Verbindung zur Verwendung bei der Prävention und/oder Reduktion von Ischämie-bedingtem Schaden in einem Subjekt, wobei die Verbindung ein Statin ist, wobei die Verbindung dem Subjekt durch intravenöse Verabreichung nach dem Beginn der Ischämie, aber vor der Reperfusion verabreicht werden soll, wobei die Verbindung so früh wie möglich nach dem Beginn der Ischämie verabreicht werden soll.

2. Verbindung zur Verwendung gemäß Anspruch 1, wobei ischämiebedingter Schaden der Schaden ist, der durch Ischämie während der ischämischen Periode nach dem Beginn der Ischämie, aber vor der Reperfusion verursacht wird.

3. Verbindung zur Verwendung gemäß irgendeinem der Ansprüche 1 oder 2, wobei die Verbindung in einer Einzeldosis zu verabreichen ist.

4. Verbindung zur Verwendung gemäß irgendeinem der Ansprüche 1 bis 3, wobei die Verbindung nicht mehr als 60 Minuten nach dem Beginn der Ischämie, vorzugsweise nicht mehr als 45 Minuten nach dem Beginn der Ischämie, weiter bevorzugt nicht mehr als 30 Minuten nach dem Beginn der Ischämie, weiter bevorzugt nicht mehr als 20 Minuten nach dem Beginn der Ischämie, weiter bevorzugt nicht mehr als 15 Minuten nach dem Beginn der Ischämie verabreicht werden soll.

5. Verbindung zur Verwendung gemäß irgendeinem der Ansprüche 1 bis 4, wobei die Verbindung mindestens 15 Minuten vor der Reperfusion, vorzugsweise mindestens 20 Minuten vor der Reperfusion, noch bevorzugter mindestens 30 Minuten vor der Reperfusion, noch bevorzugter mindestens 45 Minuten vor der Reperfusion verabreicht werden soll.

6. Verbindung zur Verwendung gemäß irgendeinem der Ansprüche 1 bis 5, wobei die Verbindung aus den in der folgenden Tabelle aufgeführten Verbindungen ausgewählt ist:
| | |
|---|---|
| 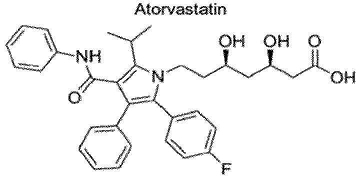 | Beta-oxidiertes Atorvastatin, ungesättigtes beta-oxidiertes Atorvastatin, beta-oxidiertes Hydroxy-Atorvastatin, 4-Hydroxy-Atorvastatin para-Hydroxy-Atorvastatinsäure, Atorvastatin-Lacton, Atorvastatin-Acyl-Glucuronid, 2-Hydroxy-Atorvastatin ortho-Hydroxy Atorvastatinsäure, 4-Hydroxy-Atorvastatin-Acyl-Glucuronid, 4-Hydroxy-Atorvastatin-Lacton und 2-Hydroxy-Atorvastatin-Lacton |
| 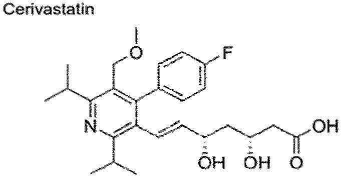 | Cerivastatin-6-O-Glucuronid, Cerivastatin-3-O-Glucuronid, Cerivastatin-4-O-Glucuronid, Cerivastatin-Lacton |
| 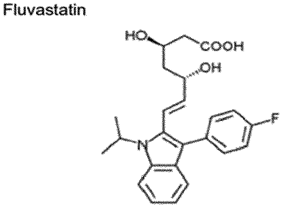 | 5-Hydroxy-Fluvstatin, 6-Hydroxy-Fluvastatin und N-Deisopropyl-Fluvastatin |
| 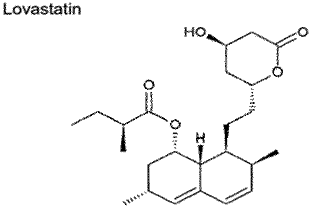 | Lovastatinsäure, 3'-Hydroxylovastatin, 6'-Exomethylenlovastatin und 6'-Beta-Hydroxylovastatin und 3"-β-Hydroxylovastatin |
| 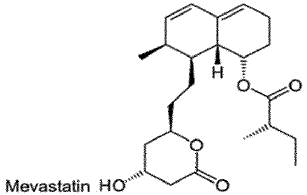 | |
| 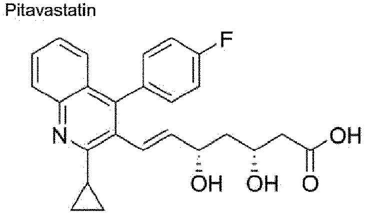 | Pitavastatin-Lacton, 3-Keto-Pitavastatin, Pitavastatin-Keto-Lacton, Hydroxy-Pitavastatin-Lacton, 3-Dehydroxy-Pitavastatin-O-Glucuronid, 5-Keto-Pitavastatin-Acyl-Glucuronid, 5-Keto-Pitavastatin-O-Glucuronid und 3-Dehydroxy-Pitavastatin-Acyl-Glucuronid |
| 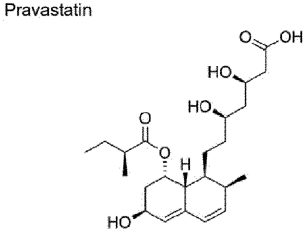 | 3" (S)-H ydroxytetran orpravastatin, 3'-alpha-5',6'-Epoxyisopravastatin, 3'-alpha-7-Hydroxyisopravastatin, 3'-Ketopravastatin-5,6-diol, 3"(S)-Hydroxypravastatin, 3'-Alphaisopravastatin, 3'-alpha-5'-beta, 6'-beta-Trihydroxypravastatin, 6'-Epipravastatin, Pravastatin-Glucuronid, Desecylpravastatin, 4'-alpha-5'-beta-Epoxypravastatin, Pravastatin-4a'-Alphaglutathion-Konjugat, Pravastatin-Lacton, Pravastatin-Sulfat |
| 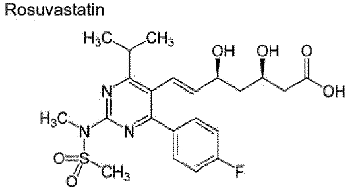 | Rosuvastatin-Lacton, N-Demethylrosuvastatin und Rosuvastatin-Acylglucuronid. |
| 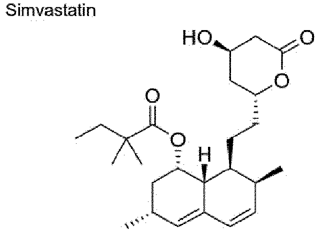 | 3',5'-Dihydrodiolsimvastatin, 6'-Beta-Hydroxymethylsimvastatin, 6'-Beta-Carboxysimvastatin, 6'-Beta-Hydroxysimvastatin, 6'-Exomethylensimvastatin, Simvastatinsäureterivastatin, Simvastatinsäureglucuronid und 3'-Hydroxysimvastatin |
wobei, vorzugsweise, die Verbindung ausgewählt ist aus der Gruppe bestehend aus Atorvastatin, der β-Hydroxysäureform von Simvastatin, Simvastatin, Cerivastatin, Fluvastatin, Lovastatin, Mevastatin, Pitavastatin, Pravastatin und Rosuvastatin, wobei, am meisten bevorzugt, die Verbindung Atorvastatin (Formel I) oder die β-Hydroxysäureform von Simvastatin der Formel II ist, wobei am meisten bevorzugt, die Verbindung Atorvastatin (Formel I) oder die β-Hydroxysäureform von Simvastatin der Formel III ist:

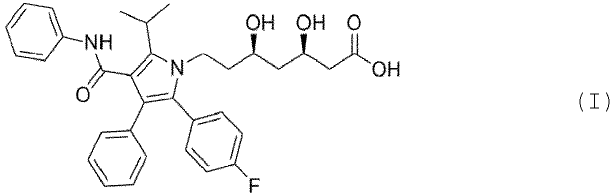

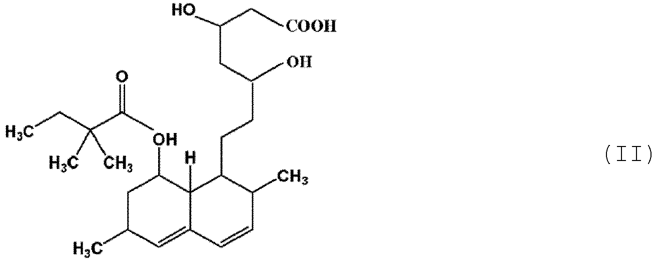

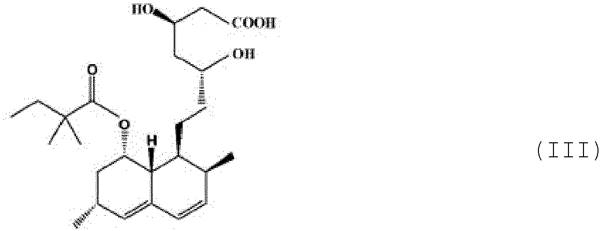

7. Verbindung zur Verwendung gemäß irgendeinem der Ansprüche 1 bis 6, wobei die Verbindung in einer Dosierung im Bereich von 0,1 bis 1 mg/kg Körpergewicht, vorzugsweise von 0,2 bis 0,8 mg/kg Körpergewicht, verabreicht werden soll.

8. Verbindung zur Verwendung gemäß irgendeinem der Ansprüche 1 bis 7, wobei die Ischämie in einem Gewebe oder Organ erzeugt wird, das aus der Gruppe ausgewählt ist, die aus Herz, Gehirn, Niere, Darm, Pankreas, Leber, Lunge, Skelettmuskel und Kombinationen davon besteht, wobei die Ischämie vorzugsweise im Herzen erzeugt wird.

9. Verbindung zur Verwendung gemäß irgendeinem der Ansprüche 1 bis 8, wobei die ischämiebedingte Schädigung auf eine Ischämie zurückzuführen ist, die durch Atherosklerose, Thrombose, Thromboembolie, Lipidembolie, Blutung, Stent, Operation, Angioplastie, Bypassoperation, Organtransplantation, stressinduzierte Kardiomyopathie (Takotsubo-Syndrom), Vasokonstriktion, ST-erhöhter Myokardinfarkt, nicht-ST-erhöhter Myokardinfarkt, instabile Angina oder eine Kombination von zwei oder mehreren dieser Faktoren ausgelöst wurde, wobei die Ischämie vorzugsweise mit ST-erhöhtem Myokardinfarkt, nicht-ST-erhöhtem Myokardinfarkt oder instabiler Angina in Zusammenhang steht.

10. Verbindung zur Verwendung gemäß irgendeinem der Ansprüche 1 bis 9, wobei der Ischämie-bedingte Schaden aus mindestens einem Schaden besteht, der aus der Gruppe ausgewählt ist, die aus Myozyten-Zelltod (vorzugsweise durch Nekrose und/oder Apoptose, besonders bevorzugt durch Nekrose), durch intrazelluläre pH-Azidifizierung aufgrund von Ischämie verursachtem Schaden und/oder durch eine Entzündungsreaktion aufgrund von Ischämie verursachtem Schaden besteht, der durch Ischämie ausgelöst und während der Reperfusion weiter verstärkt wird.

11. Verbindung zur Verwendung gemäß irgendeinem der Ansprüche 1 bis 10, wobei die ischämiebedingte Schädigung eine durch intrazelluläre Proteasen verursachte Schädigung der Myofibrillen umfasst oder daraus besteht, die eine Hyperkontraktur und/oder Kontrakturbandnekrose verursacht.

12. Verbindung zur Verwendung gemäß irgendeinem der Ansprüche 1 bis 11, wobei die Prävention/Reduktion von Ischämie-bedingtem Schaden eine Reduktion der durch Ischämie verursachten Infarktgröße, vorzugsweise eine Reduktion des Myokardschadens, umfasst oder daraus besteht,
und/oder wobei die Prävention/Reduktion von Ischämie-bedingtem Schaden zu einer Reduktion von Gewebeschäden aufgrund von durch Ischämie/Reperfusion ausgelöstem Zelltod führt, vorzugsweise aufgrund von durch Ischämie ausgelöstem Zelltod,
und/oder wobei die Prävention/Reduktion von Ischämie-bedingtem Schaden eine Prävention oder Reduktion der Entzündungsreaktion in dem ischämischen Gewebe/Organ umfasst oder daraus besteht,
und/oder wobei die Prävention/Reduktion von Ischämie-bedingtem Schaden die Reduktion der Entzündungsreaktion in Blutzellen, vorzugsweise in Leukozyten, ausgelöst durch Ischämie und/oder Reperfusion, vorzugsweise ausgelöst durch Ischämie, umfasst oder daraus besteht.

13. Verbindung zur Verwendung gemäß irgendeinem der Ansprüche 1 bis 12, wobei die Prävention/Reduktion von Ischämie-bedingtem Schaden eine Reduktion der Narbengröße in dem post-ischämischen Gewebe, vorzugsweise in post-ischämischem Herzgewebe, umfasst oder daraus besteht.

14. Verbindung zur Verwendung gemäß irgendeinem der Ansprüche 1 bis 13, wobei das Subjekt ein Mensch ist.

15. Verbindung zur Verwendung gemäß irgendeinem der Ansprüche 1 bis 14, wobei der Patient an Dyslipidämie leidet, wobei die Dyslipidämie vorzugsweise Hypercholesterinämie ist.

## Revendications

1. Composé destiné à une utilisation dans la prévention et/ou la réduction d'une lésion liée à une ischémie chez un sujet, dans lequel ledit composé est une statine, dans lequel ledit composé doit être administré audit sujet par administration intraveineuse après la survenue d'une ischémie, mais avant une reperfusion, dans lequel le composé doit être administré dès que possible après la survenue d'une ischémie.

2. Composé destiné à une utilisation selon la revendication 1, dans lequel la lésion liée à une ischémie est la lésion causée par une ischémie pendant la période ischémique après la survenue d'une ischémie, mais avant la reperfusion.

3. Composé destiné à une utilisation selon l'une quelconque des revendications 1 ou 2, dans lequel le composé doit être administré en une dose unique.

4. Composé destiné à une utilisation selon l'une quelconque des revendications 1 à 3, dans lequel le composé doit être administré pas plus de 60 minutes après la survenue d'une ischémie, de préférence pas plus de 45 minutes après la survenue d'une ischémie, plus préférentiellement pas plus de 30 minutes après la survenue d'une ischémie, plus préférentiellement pas plus de 20 minutes après la survenue d'une ischémie, plus préférentiellement pas plus de 15 minutes après la survenue d'une ischémie.

5. Composé destiné à une utilisation selon l'une quelconque des revendications 1 à 4, dans lequel le composé doit être administré au moins 15 minutes avant la reperfusion, de préférence au moins 20 minutes avant la reperfusion, plus préférentiellement au moins 30 minutes avant la reperfusion, plus préférentiellement au moins 45 minutes avant la reperfusion.

6. Composé destiné à une utilisation selon l'une quelconque des revendications 1 à 5, dans lequel le composé est sélectionné à partir des composés énumérés dans le tableau suivant :
| **Statine et formule de celle-ci Autres statines connexes** | |
|---|---|
| Atorvastatine | Atorvastatine bêta-oxydée, atorvastatine |
| 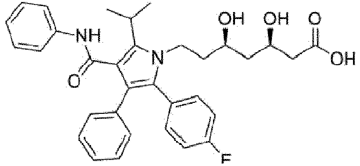 | bêta-oxydée insaturée, hydroxy-atorvastatine bêta-oxydée, acide de 4-hydroxy-atorvastatine-para-hydroxy-atorvastatine, lactone d'atorvastatine, glucuronide d'acyle d'atorvastatine, acide de 2-hydroxy-atorvastatine-orthohydroxy-atorvastatine, acyl-glucuronide de 4-hydroxy-atorvastatine, lactone de 4-hydroxy-atorvastatine et lactone de 2-hydroxy-atorvastatine. |
| Cérivastatine 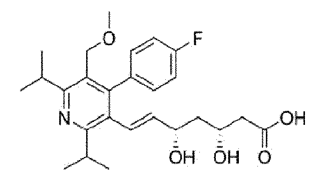 | 6-O-glucuronide de cérivastatine, 3-O-glucuronide de cérivastatine, 4-O-glucuronide de cérivastatine, lactone de cérivastatine |
| Fluvastadine 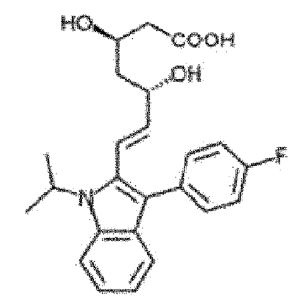 | 5-hydroxy-fluvastatine, 6-hydroxy-fluvastatine et N-déisopropyl-fluvastatine. |
| Lovastatine 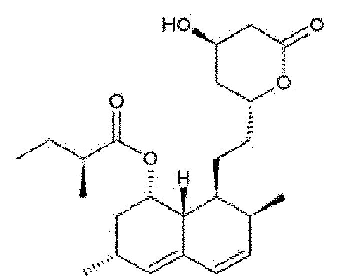 | Acide de lovastatine, 3'-hydroxylovastatine, 6'-exométhylènelovastatine et 6'-bêta-hydroxylovastatine et 3"β-hydroxylovastatine. |
| Mévastatine | |
| 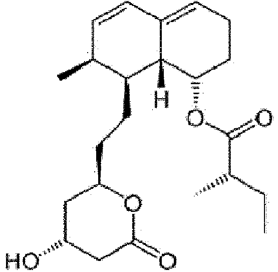 | |
| Pitavastatine 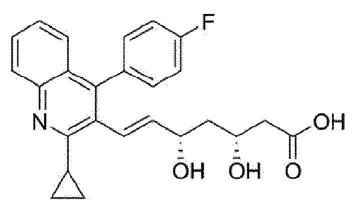 | Lactone de pitavastatine, 3-céto-pitavastatine, cétolactone de pitavastatine, lactone d'hydroxy-pitavastatine, O-glucuronide de 3-déshydroxy-pitavastatine, acyl-glucuronide de 5-céto-pitavastatine, O-glucuronide de 5-céto-pitavastatine et acyl-glucuronide de 3-déshydroxy-pitavastatine. |
| Pravastatine 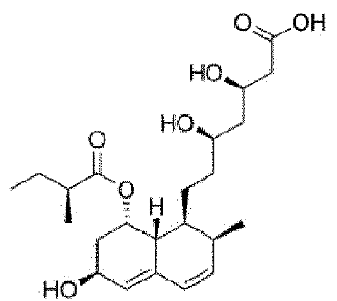 | 3"(S)-hydroxytétranorpravastatine, 3'-alpha-5',6'-époxyisopravastatine, 3'-alpha-7-hydroxyisopravastatine, 3'-cétopravastatine-5,6-diol, 3"(S)-hydroxypravastatine, 3'-alpha-isopravastatine, 3'-alpha-5'-bêta,6'-bêta-trihydroxypravatati ne, 6' -épi pravastati ne, glucuronide de pravastatine, désécylpravastatine, 4'-alpha-5'-bêta-époxypravastatine, conjugué de 4a'alpha-glutathion et de pravastatine, lactone de pravastatine, sulfate de pravastatine. |
| Rosuvastatine | Lactone de rosuvastatine, N-déméthylrosuvastatine et acyl-glucuronide de rosuvastatine. |
| 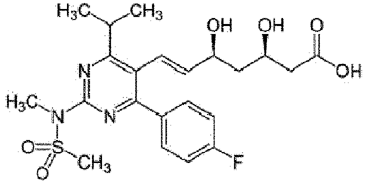 | |
| Simvastatine 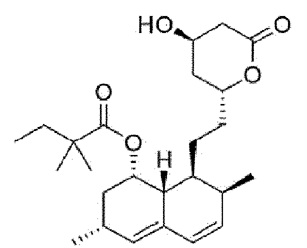 | 3',5'-dihydrodiolsimvastatine, 6'-bêta-hydroxyméthylsimvastatine, 6'-bêta-carboxysimvastatine, 6'-bêta-hydroxysimvastatine, 6'-exométhylènesimvastatine, térivastatine d'acide de simvastatine, glucuronide d'acide de simvastatine et 3'-hydroxysimvastatine |
dans lequel, de préférence, le composé est sélectionné à partir du groupe consistant en l'atorvastatine, la forme β-hydroxyacide de la simvastatine, la simvastatine, la cérivastatine, la fluvastatine, la lovastatine, la mévastatine, la pitavastatine, la pravastatine et la rosuvastatine, dans lequel, plus préférentiellement, le composé est l'atorvastatine (formule I) ou la forme β-hydroxyacide de la simvastatine de la formule II, dans lequel, le plus préférentiellement, le composé est l'atorvastatine (formule I) ou la forme β-hydroxyacide de la simvastatine de la formule III :

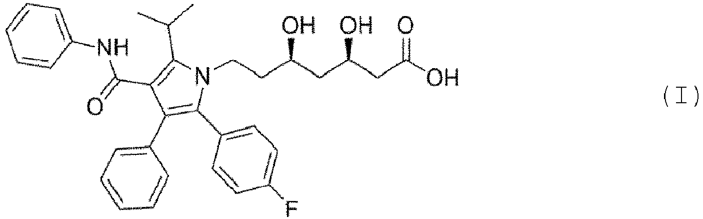

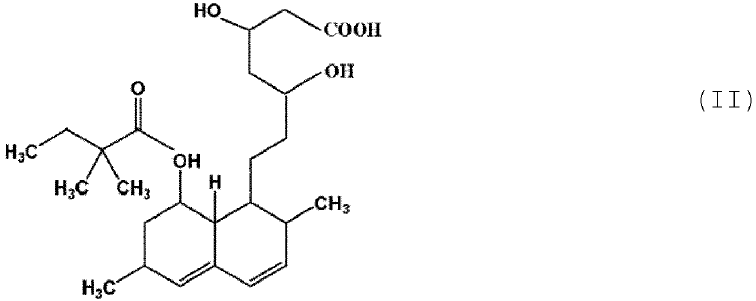

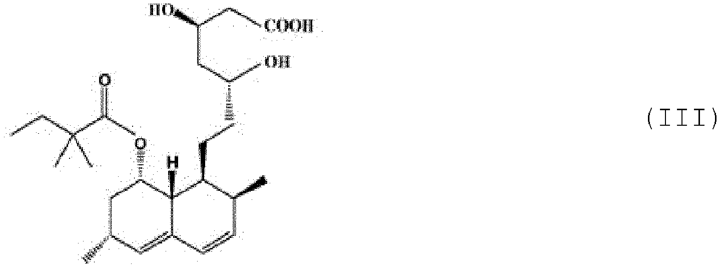

7. Composé destiné à une utilisation selon l'une quelconque des revendications 1 à 6, dans lequel le composé doit être administré à une posologie dans la plage de 0,1 à 1 mg/kg de poids corporel, de préférence de 0,2 à 0,8 mg/kg de poids corporel.

8. Composé destiné à une utilisation selon l'une quelconque des revendications 1 à 7, dans lequel l'ischémie est produite dans un tissu ou un organe sélectionné à partir du groupe consistant en le cœur, le cerveau, le rein, l'intestin, le pancréas, le foie, le poumon, le muscle squelettique et des combinaisons de ceux-ci, dans lequel, de préférence, l'ischémie est produite dans le cœur.

9. Composé destiné à une utilisation selon l'une quelconque des revendications 1 à 8, dans lequel une lésion liée à une ischémie est due à une ischémie causée par une athérosclérose, une thrombose, une thromboembolie, une embolie lipidique, une hémorragie, un stent, une chirurgie, une angioplastie, un pontage chirurgical, une greffe d'organe, une cardiomyopathie provoquée par le stress (syndrome de Takotsubo), une vasoconstriction, un infarctus du myocarde à segment ST élevé, un infarctus du myocarde à segment ST non élevé, une angine instable ou une combinaison de deux ou plus d'entre eux, dans lequel, de préférence, l'ischémie est liée à un infarctus du myocarde à segment ST élevé, un infarctus du myocarde à segment ST non élevé ou une angine instable.

10. Composé destiné à une utilisation selon l'une quelconque des revendications 1 à 9, dans lequel la lésion liée à une ischémie consiste en au moins une lésion sélectionnée à partir du groupe consistant en la mort de cellules de myocytes (de préférence par nécrose et/ou apoptose, plus préférentiellement par nécrose), une lésion causée par une acidification de pH intracellulaire due à une ischémie et/ou une lésion causée par une réponse inflammatoire due à une ischémie qui est déclenchée par une ischémie et en outre amplifiée pendant la reperfusion.

11. Composé destiné à une utilisation selon l'une quelconque des revendications 1 à 10, dans lequel la lésion liée à une ischémie comprend ou consiste en une lésion des myofibrilles causée par des protéases intracellulaires, ce qui cause une hypercontraction et/ou une nécrose de bande de contraction.

12. Composé destiné à une utilisation selon l'une quelconque des revendications 1 à 11, dans lequel la prévention/réduction d'une lésion liée à une ischémie comprend ou consiste en une réduction de la taille d'infarctus causée par une ischémie, de préférence une réduction de lésion myocardique,
et/ou dans lequel la prévention/réduction d'une lésion liée à une ischémie conduit à une réduction de lésion tissulaire due à une mort cellulaire déclenchée par ischémie/reperfusion, de préférence due à une mort cellulaire déclenchée par ischémie,
et/ou dans lequel la prévention/réduction d'une lésion liée à une ischémie comprend ou consiste en une prévention ou une réduction de la réponse inflammatoire dans le tissu/l'organe ischémique,
et/ou dans lequel la prévention/réduction d'une lésion liée à une ischémie comprend ou consiste en la réduction de la réponse inflammatoire dans des globules sanguins, de préférence dans les leucocytes, déclenchée par ischémie et/ou reperfusion, de préférence déclenchée par ischémie.

13. Composé destiné à une utilisation selon l'une quelconque des revendications 1 à 12, dans lequel la prévention/réduction d'une lésion liée à une ischémie comprend ou consiste en une réduction de la taille de cicatrice dans le tissu post-ischémique, de préférence dans du tissu cardiaque post-ischémique.

14. Composé destiné à une utilisation selon l'une quelconque des revendications 1 à 13, dans lequel le sujet est un humain.

15. Composé destiné à une utilisation selon l'une quelconque des revendications 1 à 14, dans lequel le sujet est atteint de dyslipidémie, dans lequel, de préférence, ladite dyslipidémie est l'hypercholestérolémie.
